# EUROPÄISCHE PATENTSCHRIFT

(11) **EP 2 371 807 B1**
(45) Veröffentlichungstag und Bekanntmachung des Hinweises auf die Patenterteilung: **26.07.2017**
(21) Anmeldenummer: 11160021.9
(22) Anmeldetag: 28.03.2011
(51) Int. Cl.: C07C 68/02, C07C 69/96, C01B 7/04, C01B 7/07, C08G 64/30

(54) **Verfahren zur Herstellung von Diarylcarbonaten und Polycarbonaten**
Method for manufacturing diaryl carbonates and polycarbonates
Procédé de fabrication de diarylcarbonates et de polycarbonates

(30) Priorität: 30.03.2010 EP 10158446
(43) Veröffentlichungstag der Anmeldung: 05.10.2011
(73) Patentinhaber: Covestro Deutschland AG, 51373 Leverkusen (DE)
(72) Erfinder: Ooms, Pieter, 47800, Krefeld (DE); Bulan, Andreas, 40764, Langenfeld (DE)
(74) Vertreter: Levpat

(56) Entgegenhaltungen:
- EP-A1- 0 635 477
- EP-A2- 1 112 997
- WO-A2-02/18675
- US-A- 2 362 865

## Beschreibung

Die vorliegende Erfindung betrifft ein Verfahren zur kontinuierlichen Herstellung von Diarylcarbonaten aus Phosgen und wenigstens einer Monohydroxyverbindung (Monophenol) in Gegenwart von Katalysatoren, sowie deren Verwendung zur Herstellung von Polycarbonaten. Der dabei anfallende Chlorwasserstoff wird durch thermische Oxidation zu Chlor umgesetzt, wobei das Chlor zur Herstellung des Phosgens rückgeführt wird. Insbesondere besteht das Verfahren in der Verwertung des anfallenden Chlorwasserstoffs für das Diphenylcarbonat-Herstellungsverfahren (DPC-Verfahren).

Es ist bekannt, dass Diarylcarbonate, insbesondere Diphenylcarbonat durch Phasengrenzflächenphosgenierung (Schotten-Baumann-Reaktion) von Monophenolen in einem inerten Lösungsmittel in Gegenwart von Alkali und einem Katalysator hergestellt werden können. Dabei wirkt sich die Verwendung von Lösungsmitteln und Natronlauge nachteilig aus, da durch die wässrige Lauge eine teilweise Verseifüng von Phosgen oder Chlorkohlensäureester stattfinden kann, große Mengen Kochsalz als Nebenprodukt anfallen und das Lösungsmittel und Katalysator zurückgewonnen werden müssen.

Aus dem Grund ist auch die Herstellung von Diarylcarbonaten und insbesondere Diphenylcarbonat durch Reaktion von Monophenolen und Phosgen ohne Alkali und ohne Verwendung von Lösungsmitteln in Gegenwart von einem Katalysator durch den Direktphosgenierungsprozess untersucht worden und prinzipiell in der Literatur beschrieben.

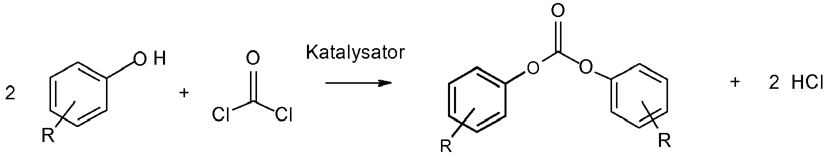

Vorschläge für Verfahren ohne Lösungsmittel mit löslichen Katalysatoren werden in US 2 837 555, 3 234 263 und 2 362 865 beschrieben.

Auch zu der Verwendung von heterogenen, nicht löslichen Katalysatoren, die eine Aufarbeitung des Reaktionsgemisches wesentlich erleichtern, wurden Vorschläge gemacht. So wird in der EP 516 355 A2 vor allem Aluminiumtrifluorid empfohlen, das auf Träger wie Alumosilikate aufgebracht wird. Die Synthese von Aluminiumfluorid ist jedoch durch die Handhabung von Fluor oder Flußsäure sehr aufwendig und teurer.

Weiter werden in der WO 91/06526 Metallsalze auf porösen Trägern als Katalysatoren für die erfindungsgemäßen Umsetzungen beschrieben. Eine vollkontinuierliche Phosgenierung von Phenol an solchen Katalysatoren ist nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

Deshalb wurden nicht-geträgerte Katalysatoren vorgeschlagen, die den großen Vorteil haben, dass man den Katalysator sehr leicht abtrennen kann und keine Verunreinigungen im rohen Reaktionsprodukt verbleiben. Die Aufarbeitung wird dadurch wesentlich vereinfacht.

So wurden Verfahren zur Herstellung von Diarylcarbonaten durch Phosgenierung von Monophenolen in Gegenwart von heterogenen Katalysatoren wie Aktivkohlen (EP 483 632), Aluminiumoxiden (EP 635 477), Alumosilikaten (EP 635 476), Metalloxiden (EP 645 364 ), Metallaten (EP 691 326), Hartstoffe (EP 722 930) und Mischhydroxiden (DE 10 2008 050 828) als auch in Gegenwart von homogenen Katalysatoren wie Metallsalzen (US 634622), aromatischen Stickstoffheterocyclen (D-OS 2 447 348) und Organophosphorverbindungen (US 5 136 077) sowohl in der Flüssigphase (EP 757 029, EP 791 574) als auch in der Gasphase (EP 808 821) beschrieben.

Nach der Synthese des Diarylcarbonats wird das Diarylcarbonat als Mischung mit dem eingesetzten Monophenol und gegebenenfalls Katalysator oder gegebenenfalls in Form seiner Lösung in dem bei der Synthese verwendeten organischen Lösungsmittel, beispielsweise Chorbenzol, abgetrennt.

Zum Erhalt des hochreinen Diarylcarbonats kann eine Reinigung durch Destillation und / oder Kristallisation erfolgen. Beispielsweise erfolgt dies durch eine oder mehrere hintereinander geschaltete Destillationskolonnen bei der gegebenenfalls das Lösungsmittel vom Diarylcarbonat abgetrennt wird.

Diese Reinigungsstufe bzw. Stufen können beispielsweise kontinuierlich so geführt werden, dass die Sumpftemperatur bei der Destillation 150°C bis 310°C, bevorzugt 160 bis 230°C beträgt. Der zur Durchführung dieser Destillation angewendete Druck beträgt dabei insbesondere 1 bis 1000 mbar, bevorzugt 5 bis 100 mbar.

Die so gereinigten Diarylcarbonate zeichnen sich durch besonders hohe Reinheit (GC >99,95%) und sehr gutes Umesterungsverhalten aus, so dass hieraus anschließend ein Polycarbonat in exzellenter Qualität hergestellt werden kann.

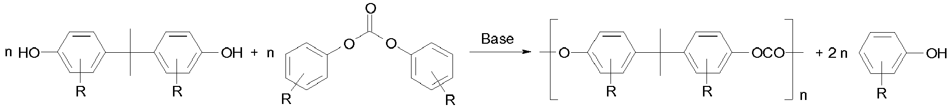

Die Verwendung der Diarylcarbonate zur Herstellung von aromatischen Oligo- / Polycarbonaten nach dem Schmelzumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964), S. 50/51 oder der US-A-5 340 905 beschrieben.

Die Nutzung des bei der Herstellung von Diphenylcarbonat durch Direktphosgenierung von Phenol gebildeten Chlorwasserstoffs kann z.B. durch Vermarktung der wässrigen Lösung (Salzsäure) oder durch Verwendung bei Synthesen anderer chemischer Produkte erfolgen. Die anfallenden Mengen Chlorwasserstoff können jedoch nicht immer vollständig vermarktet oder für andere Synthesen eingesetzt werden. Außerdem kann Chlorwasserstoff für Synthesen nur dann eingesetzt werden, wenn er zuvor entsprechend gereinigt wird. Andererseits ist die Vermarktung meist nur dann wirtschaftlich, wenn der Chlorwasserstoff bzw. die Salzsäure nicht über weite Wege transportiert werden müssen.

Eine der gängigsten Nutzungsmöglichkeiten für den anfallenden Chlorwasserstoff ist daher die Verwendung als Rohstoff bei der PVC-Herstellung, bei dem die Oxychlorierung von Ethylen mit Chlorwasserstoff zu Ethylendichlorid erfolgt. Dennoch ist diese Verfahrensweise nicht generell anwendbar, da die entsprechenden Herstellbetriebe sich meistens nicht in unmittelbarer Umgebung einer Diarylcarbonatherstellung befinden. Die Entsorgung des Chlorwasserstoffes, z.B. durch Neutralisation mit Lauge, ist aus wirtschaftlicher und ökologischer Sicht unattraktiv.

Ein Recyclingverfahren für den Chlorwasserstoff und die Rückführung des Chlors in den Diphenylcarbonat-Produktionsprozess, in dem der Chlorwasserstoff anfällt, ist daher die angestrebte Verfahrensweise.

Eine Übersicht über elektrochemische Recycling-Verfahren wird in dem Artikel "Chlorine Regeneration from Anhydrous Hydrogen Chloride" von Dennie Turin Mah, veröffentlicht in "12th International Forum Electrolysis in Chemical Industry - Clean and Efficient Processing Electrochemical Technoogy for Synthesis, Separation, Recycle and Environmental Improvement, October 11-15, 1998, Sheraton Sand Key, Clearwater Beach, FL", gegeben.

Die Rückführung von Chlorwasserstoff durch elektrochemische Oxidation unter Bildung von Chlor und Wasserstoff ist in LU 88 569 und EP 1 112 997 beschrieben. Bei dem in der EP 1 112 997 offenbarten Verfahren wird ein Diaryl-Carbonat aus einer aromatischen Monohydroxyverbindung durch Umsetzung mit Phosgen unter Einsatz einer stickstoffhaltigen heterocyclischen Verbindung als Katalysator hergestellt. Das hierbei anfallende Chlorwasserstoffgas wird von seinen Verunreinigungen befreit und entstandenes Chlorgas zurückgewonnen, welches durch Umsetzung mit Kohlenmonoxid in Phosgen umgewandelt und dem Prozess wieder zugeführt wird. Nachteilig ist die geringe Strom-ausbeute und die Produktion von Wasserstoff, der für das Polycarbonatherstellungsprozess keine Verwendung hat.

Auch die Rückführung von Chlorwasserstoff durch thermische Umsetzung mit Sauerstoff in Gegenwart von Katalysatoren ist bekannt, wie z.B. in WO 04/014845 beschrieben. Nachteilig ist daß hier die Rückgewinnung des Phosgenierungs-Katalysators Pyridin durch Behandlung mit Alkali zu einer verringerten Rückgewinnung des Chlorwasserstoffs durch die Bildung von Kochsalz führt, das entsorgt werden muß.

Ausgehend vom oben geschilderten Stand der Technik bestand die Aufgabe der vorliegenden Erfindung darin, ein Diarylcarbonat-Herstellungsverfahren bereitzustellen, welches Produkte in hoher Reinheit und guter Ausbeute liefert und eine Reduzierung der Umweltbelastung bzw. Abwasserproblematik in den Klärwerken durch maximierte Rückführung von Nebenprodukten, die aus der Polycarbonat-Produktion stammen, erreicht wird. Insbesondere sollte bei dem Recycling die Umsetzung von Chlorwasserstoff zu Chlor mit minimalem Energieeinsatz und daher resourcenschonend erfolgen.

Es wurde nun gefunden, dass sich Chlorwasserstoff besonders günstig wiederverwerten lässt, wenn dieser Chlorwasserstoff durch thermische Oxidation mit Sauerstoff nach dem Deacon-Verfahren wieder zu Chlor umgesetzt wird und zur Herstellung von Phosgen genutzt wird.

Der bei der kontinuierlichen Herstellung von Diarylcarbonaten durch Reaktion von Monophenolen und Phosgen in Gegenwart von Katalysatoren anfallende Chlorwasserstoff kann ohne aufwendige Reinigung gegebenenfalls nach einfacher Behandlung mit Aktivkohle direkt einer thermischen Oxidation zugeführt werden, wobei der Chlorwasserstoff katalytisch zu Chlor und Wasser umgesetzt wird und das Chlor zur Herstellung des Phosgens rückgeführt werden kann. Gegenstand der vorliegenden Erfindung ist demnach ein Verfahren zur Herstellung von Diarylcarbonaten ausgehend von Monophenolen und Carbonyldihalogenid, das dadurch gekennzeichnet ist, dass der dabei entstehende Halogenwasserstoff durch thermische Oxidation mit sauerstoffhaltigem Gas nach dem Deacon-Verfahren in das Halogen und dieses wiederum nach Isolierung und Reaktion mit Kohlenmonoxid zum Carbonyldihalogenid umgesetzt wird, welches anschließend wieder mit dem Monophenol zur Herstellung des Diarylcarbonats eingesetzt wird. Das Monophenol, das bei der Herstellung von lösungsmittelfreiem Polycarbonat durch Umesterung von Diarylcarbonaten und Bisphenolen freigesetzt wird, kann wiederum zur Herstellung des Diarylcarbonats verwendet werden. Weiterhin betrifft die Anmeldung ein Verfahren in dem das hergestellte Diarylcarbonat zur Herstellung von Polycarbonat verwendet wird.

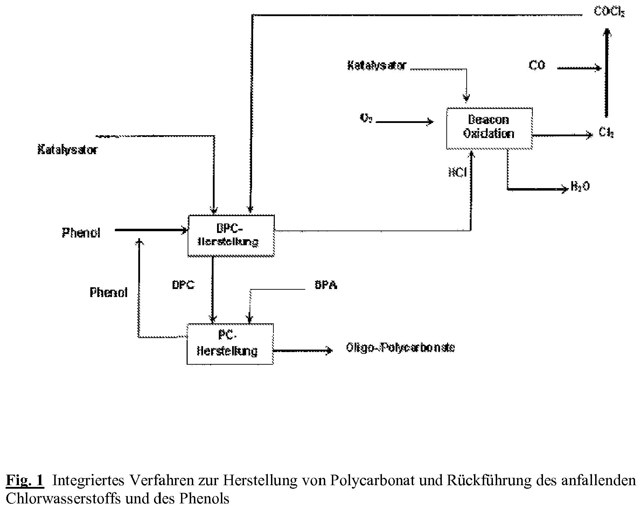

Das erfindungsgemäße Gesamtverfahren ist flexibel, einfach in der Durchführung und liefert Produkte in hoher Reinheit, die für den Gesamtprozess außerordentlich wichtig sind unter gleichzeitiger Reduzierung der Umweltbelastung durch Wiederverwendung, umfassend folgende Prozessschritte (vgl. Fig. 1):
(a) Herstellung von Phosgen durch Umsetzung von Chlor mit Kohlenmonoxid,
(b) Umsetzung des gemäß Schritt (a) gebildeten Phosgens mit wenigstens einem Monophenol in Gegenwart eines Katalysators, und gegebenenfalls organischem Lösungsmittel unter Bildung wenigstens eines Diarylcarbonats und von Chlorwasserstoff, wobei ein Metallsalz als homogener Katalysator eingesetzt wird,
(c) Abtrennung und Aufarbeitung des in Schritt (b) gebildeten Diarylcarbonats
(d) Abtrennung und gegebenenfalls Reinigung des gemäß Schritt (b) gebildeten Chlorwasserstoffs,
(e) thermische Oxidation wenigstens eines Teils des Chlorwasserstoffs aus (d) mit Sauerstoff zu Chlor unter Bildung von Wasser wobei die thermische Oxidation unter Verwendung eines oder mehrerer Katalysatoren erfolgt.
(f) Rückführung wenigstens eines Teils des gemäß Schritt (e) hergestellten Chlors in die Herstellung von Phosgen gemäß Schritt (a),
das bei der Umsetzung (e) entstehende Gasgemisch, bestehend aus den Zielprodukten Chlor und Wasser, nicht umgesetztem Chlorwasserstoff und Sauerstoff, sowie weiteren Nebenbestandteilen, insbesondere Kohlendioxid und Stickstoff, wird gegebenenfalls
1) zur Kondensation von Salzsäure abgekühlt
2) die entstandene wässrige Lösung von Chlorwasserstoff (Salzsäure) vom Gasgemisch abgetrennt,
3) die abgetrennte Salzsäure mindestens teilweise einer elektrochemischen Oxidation zugeführt, in der wenigstens ein Teil der wässrigen Salzsäure zu Chlor oxidiert wird,
4) das in Schritt 3) anfallende Chlorgas gegebenenfalls dem in Schritt (e) anfallenden Gasgemisch zugegeben,
5) die im Gasgemisch aus den Schritten 3) und (e) vorhandenen Reste an Wasser, insbesondere durch Wäsche mit Schwefelsäure, entfernt,
6) das entstehende chlorreiche Gasgemisch von Sauerstoff und gegebenenfalls von Nebenbestandteilen befreit.

Die Durchführung der thermische Oxidation mit Sauerstoff nach dem Deacon-Verfahren führt zu einer erhöhten Wirtschaftlichkeit im Vergleich mit einer klassischen Elektrolyse, die zudem neben Chlor noch Wasserstoff produziert, der in eine Diaryl- oder Polycarbonatherstellung keine Verwendung hat.

In einer besonders bevorzugten Ausführungsform wird wenigstens ein Teil des gemäß Schritt (c) hergestellten Diarylcarbonats mit einem Bisphenol zum Oligo-/Polycarbonat und dem Monophenol umgesetzt (die sogenannte Umesterungsreaktion). Das bei der Umesterung entstehende Monophenol kann in einer weiteren bevorzugten Ausführungsform wiederum in Schritt (b) eingesetzt werden.

Das erfindungsgemäße Verfahren ist ein Verfahren zur Herstellung von Diarylcarbonaten und die thermische Oxidation des Chlorwasserstoffs mit Sauerstoff zur Rückgewinnung von Chlor für die Synthese von Phosgen als Ausgangsprodukt für die Diarylcarbonatherstellung.

Im ersten Schritt (a) des erfindungsgemäßen Verfahrens erfolgt die Herstellung von Phosgen durch Umsetzung von Chlor mit Kohlenmonoxid. Die Synthese von Phosgen ist hinlänglich bekannt und ist z.B. in Ullmanns Enzyklopädie der industriellen Chemie, 3. Auflage, Band 13, Seite 494-500 dargestellt. Im technischen Maßstab wird Phosgen überwiegend durch Umsetzung von Kohlenmonoxid mit Chlor bevorzugt an Aktivkohle als Katalysator hergestellt. Die stark exotherme Gasphasenreaktion erfolgt bei Temperaturen von mindestens 250°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO 03/072237 beschrieben, oder durch Siedekühlung über einen Sekundärkühlkreislauf unter gleichzeitiger Nutzung der Reaktionswärme zur Dampferzeugung, wie z.B. in US 4 764 308 offenbart.

Aus dem gemäß Schritt (a) gebildeten Phosgen wird durch Umsetzung mit wenigstens einem Monophenol in einem nächsten Verfahrensschritt (b) wenigstens ein Diarylcarbonat gebildet. Der Verfahrensschritt (b) wird nachfolgend auch als Phosgenierung bezeichnet. Die Umsetzung erfolgt unter Bildung von Chlorwasserstoff als Nebenprodukt.

Die Synthese von Diarylcarbonaten ist ebenfalls aus dem Stand der Technik hinlänglich bekannt, wobei in der Regel das Monophenol in einem stöchiometrischen Überschuss, bezogen auf das Phosgen, eingesetzt wird. Üblicherweise findet die Phosgenierung gemäß (b) in der Flüssigphase statt, wobei das Phosgen und das Monophenol in der Schmelze, gegebenenfalls in einem Lösungsmittel gelöst sein können. Bevorzugte Lösungsmittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Toluol, Xylole und das Monophenol selbst.

Besonders bevorzugt ist das geschmolzene Monophenol als Lösungsmittel.

In einer anderen, bevorzugten Ausführungsform findet die Phosgenierung in der Gasphase statt. Die Gasphasenphosgenierung ist z.B. in US 5 831 111 beschrieben.

Für das erfindungsgernäße Verfahren geeignete Monophenole sind Phenole der Formel (I) 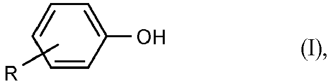 worin
- R: Wasserstoff, Halogen oder ein verzweigter oder unverzweigter C₁- bis C₉-Alkylrest, C₁- bis C₉-Alkoxyrest oder C₁- bis C₉-Alkoxycarbonylrest ist.

Bevorzugt sind also Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol, 2,4,6-Tribromphenol, Anisol und Salicylsäuremethyl oder -phenylester. Besonders bevorzugt ist Phenol.

Für das erfindungsgemäße Verfahren werden homogene Katalysatoren eingesetzt.

Als homogene Katalysatoren werden Metallsalze oder deren Alkylate eingesetzt.

Es können ein oder mehrere, aktivierte oder nicht-aktivierte Katalysatoren eingesetzt werden. Andere geeignete Katalysatoren für Schritt b) des hierin beschriebenen Verfahrens sind:
a) Aktivkohlen mit nach der BET-Methode bestimmten Oberflächen von 200 bis 3000 m2/g, die aus Sägemehl und anderen Holzabfällen, Stroh, Kohlensorten, Nußschalen, Mineralölteeren, Lignin, Polysacchariden, Polyacrylnitril, Knochen, Torf oder Koksprodukte aus Braun oder Steinkohlen, bevorzugt aus Holz, Cellulose, Lignin, Bitumen- oder Braunkohle, Torf oder Steinkohlenkoks hergestellt wurde.
b) Alumosilikaten, ausgewählt aus der Gruppe der Zeolithe der allgemeinen Formel

   M_{2/n} O. x SiO₂. Al₂O₃. y H₂O

   in welcher
   M für Kationen wie Protonen oder Metallkationen des Periodensystems der Elemente nach Mendelejew steht,
   n für die Wertigkeit des Kations steht,
   x für das Mol-Verhältnis SiO₂.Al₂O₃ steht, wobei
   x eine Zahl von 1,0-50,0 sein kann, und
   y für eine Zahl von 0 - 9 steht,
   oder zeolith-ähnliche Verbindungen wie ALPO's und SAPO's oder Schichtsilikate des Kaolin-, Serpentin-, Montmorillonit- und Bentonit-Typs oder "pillared clays" oder SiO₂/Al₂O₃-Fällungskatalysatoren,
c) Aluminiumoxide oder y-Aluminiumoxide mit nach der BET-Methode bestimmten Oberflächen von 2 bis 500 m²/g,
d) Oxid(e) von Metallen der Gruppe IVB des periodischen Systems wie beispielsweise Oxide des Titans, Zirkoniums oder Hafniums mit nach der BET-Methode bestimmten Oberflächen von 2 bis 500 m²/g,
e) Metallate der allgemeinen Formel

   AₓByO_{z}

   in welcher
   A für ein mono-, di und/oder trivalentes Metallkation steht und
   B für ein tri-, tetra-, penta- undloder hexavalentes Metallkation steht und
   x für eine Zahl von 1 bis 4 steht und
   y für eine Zahl von 1 oder 2 steht und
   z für eine Zahl von 3, 6, 7 oder 9 steht,
f) Hartstoffen mit metallartigen Eigenschaften (Keramikvorprodukte) der allgemeinen Formel

   AₓB_{y}C_{z}D_{w}

   in welcher
   A für ein Element aus den Gruppen 3 bis 10, 13 und 14 des periodischen Systems der Elemente (IUPAC Notation) steht und
   B für ein Element aus den Gruppen 13, 14, 15 und 16 mit Ausnahme von Sauerstoff steht,
   C für ein Element aus den Gruppen 14 und 15 steht und
   D für ein Element aus den Gruppen 14 und 15 steht und
   x für eine Zahl von 1 bis 4 steht und
   y für eine Zahl von 1 bis 4 steht und
   z für eine Zahl von 0 bis 4 steht und
   w für eine Zahl von 0 bis 4 steht
   wobei A, B, C und D jeweils aus verschiedenen Gruppen oder bei gleicher Gruppe aus verschiedenen Perioden stammen, mit der Maßgabe, dass A verschieden von Aluminium ist, wenn B Kohlenstoff ist und gleichzeitig z und w gleich 0 sind,
g) Mischhydroxide der allgemeinen Formel (III)

   [M(II)₁₋ₓ M(III)ₓ M(IV)_{y} (OH)₂] Aⁿ⁻_{z/n}· m H₂O (III),

   in welcher
   M (II) für ein divalentes Metallkation steht und
   M (III) für ein trivalentes Metallkation steht und
   M(IV) für ein tetravalentes Metallkation steht und
   x für eine Zahl von 0,1 bis 0,5 steht und
   y für eine Zahl von 0 bis 0,5 steht
   z für 1 + y steht und
   m für eine Zahl von 0 bis 1 steht
   A für ein anorganisches Anion wie OH⁻, NO₃⁻, CO₃²⁻, SO₄²⁻, CrO₄²⁻ oder Cl⁻ steht.
   n für 1 bis 2 steht
h) Metallsalze, -halogenide, -alkylate und -phenolate wie z.B. AlCl₃, AlF₃, ZrCl4, TiCl₄, VCl₃, VCl₄ oder Ti(OC₆H₅)₄,
i) Organophosphorverbindungen, homogen oder gegebenenfalls polymergebunden.

Gegebenenfalls, jedoch nicht erfindungsgemäß, können die homogenen Katalysatoren auf einem inerten Träger aufgebracht werden, wie in JP 695219, WO 91/06526, US 5 424 473 und EP 516 355 beschrieben.

Bevorzugt sind Titanchlorid, Zirkonchlorid, Aluminiumchlorid und Titanphenolat, die als homogene Katalysatoren eingesetzt werden.

Besonders bevorzugt sind Aluminiumoxid und TiCl₄.

Der Katalysator wird in Mengen von 0,5 bis 100 Gew.-% bezogen auf die Menge an Monohydroxyverbindung (Monophenol), bei nicht vollkontinuierlicher Fahrweise bzw. mit Belastungen von 0,1 bis 20 g Monohydroxyverbindung (Monophenol) pro g Katalysator pro Stunde bei vollkontinuierlicher Fahrweise, eingesetzt.

Phosgen kann in dem Verfahrensschritt (b) flüssig, gasförmig oder gegebenenfalls gelöst in einem inerten Lösungsmittel eingesetzt werden.

In dem neuen Verfahren in Schritt (b) gegebenenfalls verwendbare inerte organische Lösungsmittel sind beispielsweise Toluol, Chlorbenzol, Dichlorbenzol und Chlortoluol, bevorzugt ist Phenol selbst.

Wird ein Phosgenierung in der Gasphase gewählt, so erfolgt die Umsetzung oberhalb der Siedetemperatur des Phenols, bevorzugt innerhalb einer mittleren Kontaktzeit von 0,5 bis 5 Sekunden und bei Temperaturen von 180 bis 500°C.

Bei der Phosgenierung in der Flüssigphase werden üblicherweise Temperaturen von 20 bis 240°C und Drücke von 1 bis ca 50 bar eingesetzt. Die Phosgenierung in der Flüssigphase kann einstufig oder mehrstufig durchgeführt werden, wobei im allgemeinem Phenol im stöchiometrischen Überschuss eingesetzt werden kann.

Dabei können das Phenol und das Phosgen über ein statisches Mischelement vereinigt, in Gegen- oder Gleichstrom beispielsweise über ein Festbett (heterogen) oder in eine oder mehrere Reaktivdestillationskolonnen oder Blasensäulen (homogen) geführt werden, wo das Gemisch zum gewünschten Diarylcarbonat und Chlorwasserstoff abreagiert. Neben Reaktionstürmen, die mit geeigneten Mischelementen versehen sind, können auch Reaktionsbehälter mit Rührvorrichtung eingesetzt werden. Außer statischen Mischelementen können auch spezielle dynamische Mischelemente Anwendung finden. Geeignete statische und dynamische Mischelemente sind aus dem Stand der Technik bekannt.

Nach der Phosgenierung gemäß Schritt (b) erfolgt erfindungsgemäß in Schritt (c) die Abtrennung der bei der Phosgenierung gebildeten Diarylcarbonate. Dies geschieht dadurch, dass zunächst das Reaktionsgemisch der Phosgenierung in einen flüssigen und einen gasförmigen Produktstrom in einer dem Fachmann bekannten Weise aufgetrennt wird. Der flüssige Produktstrom enthält im Wesentlichen das Diarylcarbonat, gegebenenfalls das Lösungsmittel sowie einen geringen Teil an nicht umgesetztem Phosgen. Der gasförmige Produktstrom besteht im Wesentlichen aus Chlorwasserstoffgas, nicht umgesetztes Phosgen, sowie geringfügigen Mengen an gegebenenfalls Lösungsmittel und Inertgasen, wie zum Beispiel Stickstoff und Kohlenmonoxid. Ferner wird der Flüssigstrom gemäß Schritt (c) anschließend einer Aufarbeitung zugeführt, vorzugsweise einer destillativen Aufarbeitung, wobei nacheinander Phosgen sowie gegebenenfalls das Lösungsmittel abgetrennt werden. Gegebenenfalls erfolgt gemäß Schritt (c) außerdem eine weitere Aufarbeitung der gebildeten Diarylcarbonate. Dies geschieht beispielsweise, indem das erhaltene Diarylcarbonat in einer dem Fachmann bekannten Weise gereinigt wird durch Destillation oder Kristallisation.

Die in Schritt (d) gegebenenfalls notwendige Reinigung des Chlorwasserstoffs kann dadurch bedingt sein, dass der bei der Reaktion von Phosgen mit einem Phenol erhaltene Chlorwasserstoff im Allgemeinen organische Bestandteile enthält, welche bei der thermischen Oxidation von Chlorwasserstoff gemäß Schritt (e) stören können, so dass bei einer Desaktivierung des katalytisch aktiven Materials durch Kontamination oder Beschädigung, der Katalysator ausgetauscht werden muss. Zu diesen organischen Bestandteilen zählen beispielsweise Phosgen, das Monophenol oder die gegebenenfalls bei der Diarylcarbonatherstellung eingesetzten Lösungsmittel wie Chlorbenzol, o-Dichlorbenzol oder p-Dichlorbenzol. Erfolgt die thermische Oxidation nach dem Deacon-Verfahren in der Gasphase in Gegenwart von Katalysatoren, so könnte der Katalysator durch diese organischen Bestandteile in seiner Funktion beeinträchtigt werden. Die Verunreinigungen können sich auch auf dem Katalysator ablagern und dadurch die Aktivität verschlechtern, was einen Verringerung der Chlor-Ausbeute zur Folge hat.

Dementsprechend erfolgt in einem weiteren Verfahrensschritt (d) die Abtrennung des bei der Phosgenierung gemäß Schritt (b) erzeugten Chlorwasserstoffs aus dem gasförmigen Produktstrom. Der gasförmige Produktstrom der bei der Abtrennung des Diphenylcarbonats gemäß Schritt (c) erhalten wird, wird gemäß Schritt (d) so behandelt, dass das Phosgen abgetrennt wird und der Chlorwasserstoff, gegebenenfalls nach Reinigung, einer thermischen Oxidation gemäß Schritt (e) zugeführt werden kann.

Die Abtrennung des Chlorwasserstoffs gemäß Schritt (d) erfolgt zunächst, indem Phosgen aus dem gasförmigen Produktstrom abgetrennt wird. Die Abtrennung des Phosgens gelingt durch Verflüssigung von Phosgen, beispielsweise an einem oder mehreren in Reihe geschalteten Kondensatoren. Die Verflüssigung erfolgt vorzugsweise bei Temperaturen im Bereich von -15 bis -40°. Durch diese Tiefkühlung können außerdem gegebenenfalls Teile der organischen Verunreinigungen, wie z.B. Monophenol aus dem gasförmigen Produktstrom entfernt werden.

Zusätzlich oder alternativ kann das Phosgen mit einem kalten Lösungsmittel oder Lösungsmittel-Phosgen-Gemisch aus dem Gasstrom in einer oder mehreren Stufen ausgewaschen werden. Als Lösungsmittel hierfür eignen sich beispielsweise die gegebenenfalls bereits in der Phosgenierung eingesetzten Lösungsmittel Chlorbenzol und o-Dichlorbenzol. Die Temperatur des Lösungsmittels oder Lösungsmittel-Phosgen-Gemisches hierfür liegt im Bereich von -15 bis -46°C.

Das aus dem gasförmigen Produktstrom abgetrennte Phosgen kann wieder der Phosgenierung gemäß Schritt (b) zugeführt werden.

Gegebenenfalls erfolgt anschließend gemäß Schritt (d) eine Reinigung des Chlorwasserstoffs, um den Anteil an organische Verunreinigungen wie z.B. unumgesetztes Monophenol zu verringern.

Dies kann beispielsweise mittels Ausfrieren erfolgen, indem in Abhängigkeit von den physikalischen Eigenschaften des Monophenols der Chlorwasserstoff zum Beispiel durch eine oder mehrere Kühlfallen geleitet wird.

In einer bevorzugten Ausführungsform der gemäß Schritt (d) gegebenenfalls vorgesehenen Reinigung des Chlorwasserstoffs werden zwei in Serie geschaltete Wärmetauscher von dem Chlorwasserstoffstrom durchströmt, wobei das abzutrennende Monophenol in Abhängigkeit des Festpunktes zum Beispiel bei -40°C ausgefroren wird. Die Wärmetauscher werden wechselweise betrieben, wobei im jeweils zuerst durchströmten Wärmetauscher der Gasstrom das zuvor ausgefrorene Monophenol auftaut. Das Monophenol kann wieder für die Herstellung des Diarylcarbonats eingesetzt werden. Im nachgeschalteten zweiten Wärmetauscher, der mit einem üblichen Wärmeträgermedium für Kältemaschinen, z.B. eine Verbindung aus der Reihe der Frigene, beaufschlagt ist, wird das Gas unter den Festpunkt des Monophenols abgekühlt, sodass dieses auskristallisiert. Nach abgeschlossenem Auftau- und Kristallisationsvorgang werden der Gasstrom und der Kühlmittelstrom umgeschaltet, sodass sich die Funktion der Wärmetauscher umkehrt. Der chlorwasserstoffhaltige Gasstrom kann auf diese Weise auf vorzugsweise maximal 500 ppm, besonders bevorzugt maximal 50 ppm, ganz besonders bevorzugt auf maximal 20 ppm Monophenolgehalt abgereichert werden.

Alternativ kann die Reinigung des Chlorwasserstoffs in zwei in Serie geschaltete Wärmetauscher gemäß US 6 719 957 erfolgen. Dabei wird der Chlorwasserstoff auf einen Druck von 5 bis 20 bar, bevorzugt 10 bis 15 bar, verdichtet und der komprimierte gasförmige Chlorwasserstoff mit einer Temperatur von 20 bis 60°C, bevorzugt 30 bis 50°C, einem ersten Wärmeaustauscher zugeführt. In diesem wird der Chlorwasserstoff mit einem kalten Chlorwasserstoff einer Temperatur von -10 bis -30°C, der aus einem zweiten Wärmetauscher stammt, gekühlt. Dabei kondensieren organische Bestandteile, die einer Entsorgung oder Wiederverwertung zugeführt werden können. Der in den ersten Wärmetauscher geleitete Chlorwasserstoff verlässt diesen mit einer Temperatur von -20 bis 0°C und wird in dem zweiten Wärmetauscher auf eine Temperatur von - 10 bis -30°C gekühlt. Das im zweiten Wärmetauscher anfallende Kondensat besteht aus weiteren organischen Bestandteilen sowie geringen Mengen Chlorwasserstoff. Zur Vermeidung eines Chlorwasserstoffverlustes wird das aus dem zweiten Wärmetauscher ablaufende Kondensat einer Abtrenn- und Verdampfereinheit zugeführt. Dies kann beispielsweise eine Destillationskolonne sein, in der aus dem Kondensat der Chlorwasserstoff ausgetrieben und in den zweiten Wärmetauscher zurückgeführt wird. Es ist auch möglich, den ausgetriebenen Chlorwasserstoff in den ersten Wärmetauscher zurückzuführen. Der in dem zweiten Wärmetauscher abgekühlte und von organischen Bestandteilen befreite Chlorwasserstoff wird mit einer Temperatur von -10 bis -30°C in den ersten Wärmetauscher geleitet. Nach Erwärmung auf 10 bis 30°C verlässt der von organischen Bestandteilen befreite Chlorwasserstoff den ersten Wärmetauscher.

In einem alternativen Verfahren erfolgt die gemäß Schritt (d) gegebenenfalls vorgesehene Reinigung des Chlorwasserstoffs mittels Adsorption von organischen Verunreinigungen, wie Monophenolreste, an Aktivkohle. Dabei wird beispielsweise der Chlorwasserstoff nach Entfernung von überschüssigem Phosgen bei einem Druck von 0 bis 5 bar, vorzugsweise von 0,2 und 2 bar, über oder durch eine Aktivkohleschüttung geleitet. Die Strömungsgeschwindigkeiten und Verweilzeiten werden dabei in einer dem Fachmann bekannten Weise dem Gehalt an Verunreinigungen angepasst. Die Adsorption von organischen Verunreinigungen ist ebenso an anderen geeigneten Adsorptionsmitteln möglich, so z.B. an Zeolithen.

In einem weiteren alternativen Verfahren kann für die gemäß Schritt (d) gegebenenfalls vorgesehene Reinigung des Chlorwasserstoffs eine Destillation des Chlorwasserstoffes vorgesehen sein. Diese erfolgt nach Kondensation des gasförmigen Chlorwasserstoffes. Bei der Destillation des kondensierten Chlorwasserstoffes wird der gereinigte Chlorwasserstoff als Kopfprodukt der Destillation entnommen, wobei die Destillation unter dem Fachmann bekannte, für eine solche Destillation übliche Bedingungen von Druck, Temperatur u.ä. erfolgt.

Nach Reinigung wird der Chlorwasserstoff mit Sauerstoff in Gegenwart eines Kata-lysators umgesetzt.

Die Reaktion kann in Gegenwart von Katalysatoren bei einer Temperatur von ca. 250 bis 450°C durchgeführt werden. Der übliche Reaktionsdruck liegt im Bereich von 1 bis 10 bar. Es sind verschiedene Ausführungsformen des Verfahrens, das allgemein als Deacon-Verfahren bekannt ist, beschrieben: Shell-Chlor-Verfahren, MT-Chlor-Verfahren, KEL-Chlor-Verfahren, Carrier Catalyst Process und Sumitomo-Chlor-Verfahren.

Geeignete Katalysatoren für das Deacon-Verfahren enthalten Übergangsmetallverbindungen wie Kupfer- und Rutheniumverbindungen oder auch Verbindungen anderer Edelmetalle wie Gold und Palladium. Solche Katalysatoren sind beispielsweise in den Schriften DE 1 567 788 A1, EP 251 731 A2, EP 936 184 A2, EP 761 593 A1, EP 711 599 A1 und DE 102 50 131 A1 beschrieben. Die Katalysatoren sind in der Regel auf einem Träger aufgebracht. Diese Träger bestehen beispielsweise aus Siliciumdioxid, Aluminiumoxid, Titandioxid oder Zirkonoxid.

Die Deacon-Verfahren werden in der Regel in Wirbelbettreaktoren oder Festbettreaktoren, z.B. in Rohrbündelreaktoren, durchgeführt. Chlorwasserstoff wird vor der Reaktion von Verunreinigungen befreit um eine Vergiftung der eingesetzten Katalysatoren zu vermeiden. Die Reaktion kann nahezu isotherm oder nahezu adiabat betrieben werden.

Alternativ dazu sind Verfahren bekannt, bei denen die Reaktion von Chlorwasserstoff mit Sauerstoff nichtthermisch aktiviert wird. Solche Verfahren sind in "W. Stiller, Nichtthermische aktivierte Chemie, Birkhäuser Verlag, Basel, Boston, 1987, S. 33-34, S. 45-49, S122-124, S. 138-145", beschrieben. Spezielle Ausführungsformen sind beispielsweise in den Schriften RU 2253607, JP-A-59073405, DD-A-88 309, SU 1801943 A1 offenbart. Unter nichtthermisch aktivierten Reaktionen versteht man beispielsweise Anregungen der Reaktion mit folgenden Mitteln oder Verfahren:
- energiereiche Strahlung, z.B. Laserstrahlung, photochemische Strahlungsquellen, UV-Strahlung, Infrarotstrahlung u. a.
- einen Niedertemperaturplasma, z.B. durch elektrische Entladung erzeugt
- Magnetfeldanregung
- Tribomechanische Aktivierung, z.B. Anregung durch Stoßwellen
- ionisierende Strahlung, z.B. Gamma- und Röntgenstrahlung, α- und β-Strahlen aus Kernzerfällen, hochenergetische Elektronen, Protonen, Neutronen und schwere Ionen
- Mikrowellenbestrahlung

Das sauerstoffhaltige Gas wird sowohl bei der thermischen als auch bei der nichtthermischen aktivierten Umsetzung von Chlorwasserstoff mit Sauerstoff in der Regel als reines Gas mit einem O₂-Gehalt von > 98 Vol- %, eingesetzt. Geringere Anteile an Sauerstoff sind aber auch möglich. Neben Sauerstoff kann das sauerstoffhaltige Gas auch beispielsweise Stickstoff oder Kohlendioxid enthalten.

Allen bekannten Verfahren ist gemeinsam, dass bei der Reaktion von Chlorwasserstoff mit Sauerstoff ein Gasgemisch erhalten wird, das neben dem Zielprodukt Chlor auch Wasser, nicht umgesetzten Chlorwasserstoff und Sauerstoff sowie weitere Nebenbestandteile wie Kohlendioxid enthält. Zur Gewinnung von reinem Chlor wird das Produktgasgemisch nach der Reaktion soweit abgekühlt, dass Reaktionswasser und Chlorwasserstoff in Form von konzentrierter Salzsäure auskondensieren. Die entstehende Salzsäure wird abgetrennt und das verbleibende gasförmige Reaktionsgemisch wird durch Wäsche mit Schwefelsäure oder anderen Methoden wie Trocknung mit Zeolithen von restlichem Wasser befreit.

Die abgetrennte Salzsäure wird vorzugsweise einer Desorptionsstufe zugeführt, in der gasförmiger Chlorwasserstoff wieder freigesetzt wird. Dieser gasförmige Chlorwasserstoff kann teilweise oder vorzugsweise vollständig in die Umsetzung von Chlorwasserstoff mit Sauerstoff zurückgeführt werden. Die in der Desorptionsstufe anfallende verdünnte Salzsäure kann in die Salzsäure-Kondensationsstufe zurückgeführt werden. Hier dient die verdünnte Salzsäure als Absorptionsmittel für den abzutrennenden gasförmigen Chlorwasserstoff. Ein derartiges Vorgehen ist beispielsweise in der Schrift DE 102 35 476 A1 beschrieben. Alternativ dazu kann die abgetrennte Salzsäure auch einer anderen Verwertung zugeführt werden.

Das von restlichem Wasser befreite chlorhaltige Reaktionsgasgemisch wird in einer bevorzugten Aufführungsform anschließend verdichtet, wobei Sauerstoff und andere Gasbestandteile in der Gasphase bleiben und vom verflüssigten Chlor abgetrennt werden können. Derartige Verfahren zur Gewinnung von reinem Chlor aus Gasgemischen sind beispielsweise in den Offenlegungsschriften DE 195 35 716 A1 und DE 102 35 476 A1 beschrieben. Das nunmehr gereinigte Chlor kann dann der Verwendung, beispielsweise der Herstellung von Phosgen zugeführt werden.

Erfindungsgemäß wird im nächsten Verfahrensschritt (f) wenigstens ein Teil des gemäß Schritt (e) hergestellten Chlors in die Herstellung von Phosgen gemäß Schritt (a) rückgeführt. Vor der Rückführung wird das Chlor vorzugsweise in einer ein- oder mehrstufigen Kühlung mittels eines Kühlaggregats, z.B. eines Röhrenwärmetauscher, abgekühlt und getrocknet. Die Trocknung kann zum Beispiel mit Hilfe eines geeigneten Trocknungsmittels in einer mit Stoffaustauschelementen bestückten Absorptionskolonne erfolgen. Ein geeignetes Trocknungsmittel kann, wie z.B. in DE 10 235 476 A beschrieben, neben Molsieben oder hygroskopischen Adsorbentien z.B. Schwefelsäure sein. Die Trocknung kann ein- oder mehrstufig erfolgen. Bevorzugt erfolgt die Trocknung zweistufig, indem das zu trocknende Chlor in einer ersten Stufe mit einer Schwefelsäure geringerer Konzentration, vorzugsweise 70 bis 80%, besonders bevorzugt 75 bis 80%, in Kontakt gebracht wird. In einer zweiten Stufe wird die Restfeuchte mittels einer höher konzentrierten Schwefelsäure von bevorzugt 88 bis 96%, besonders bevorzugt 92-96%, aus dem Chlor entfernt. Das auf diese Art getrocknete Chlor mit einer Restfeuchte von bevorzugt maximal 100 ppm, besonders bevorzugt maximal 20 ppm kann durch einen Tröpfchenabscheider geleitet werden, um gegebenenfalls darin noch enthaltene Schwefelsäuretröpfchen zu entfernen.

Nach Herstellung einer wässrigen Chlorwasserstofflösung gemäß Schritt 1) und 2) sowie gegebenenfalls nach einer Reinigung der wässrigen Chlorwasserstofflösung wird die Salzsäure einer Elektrolysezelle zugeführt. Die elektrochemische Oxidation der Salzsäure gemäß Schritt 3) erfolgt nach dem Membranverfahren.

Gemäß WO 97/24320 sind u.a. sogenannte Festelektrolytsysteme, z.B. Nafion^{®}-Membrane, eingesetzt werden, wobei die Kathode auf einer Seite der Ionenaustauschermembran anliegt. Die Kathode ist z.B. eine Gasdiffusionselektrode. Das katalytisch aktive Material der Kathode ist in die Ionenaustauschermembran eingearbeitet oder kann auf die Ionenaustauschermembran aufgebracht sein.

Die elektrochemische Oxidation einer wässrigen Lösung von Chlorwasserstoff unter Verwendung einer Gasdiffusionselektrode als Kathode ist z.B. in WO 00/73538 und WO 02/18675 beschrieben. Dabei wird als Katalysator für die Sauerstoffreduktion an der Kathode Rhodiumsulfid eingesetzt. Gemäß WO 02/18675 ist dieser Katalysator weitgehend beständig gegen organische Bestandteile, die als Verunreinigungen in der Salzsäure vorliegen können und z.B. aus vorgeschalteten Syntheseschritten stammen. Die organischen Bestandteile gelangen über die Ionenaustauschermembran aus dem Anodenraum in den Kathodenraum.

Die elektrochemische Oxidation der Salzsäure gemäß Schritt (3) kann nach dem Membranverfahren in einer Zweiraum-Elektrolysezelle, bestehend aus Anodenraum und Kathodenraum, oder in einer Dreiraum-Elektrolyszelle, bestehend aus Anoden-raum, Kathodenraum und einem Elektrolytraum zwischen Anoden- und Kathodenraum, durchgeführt werden. Bevorzugt wird eine Zweiraum-Elektrolysezelle gewählt. Beim Membranverfahren ist der Anodenraum von dem Kathodenraum durch eine Ionenaustauschermembran (nachfolgend vereinfacht auch als Membran bezeichnet), insbesondere eine Kationenaustauschermembran, getrennt. Der Abstand der Elektroden (Anode und Kathode) von der Membran beträgt vorzugsweise 0 bis 3 mm, besonders bevorzugt 0 bis 2 mm. Geeignete Ionenaustauschermembrane sind kommerziell erhältlich, wie z.B. einschichtige Ionenaustauschermembrane mit Sulfonsäuregruppen. Beispielsweise kann eine Membran vom der Fa. DuPont Typ Nafion^{®} 117 eingesetzt werden.

Bei der Elektrolyse von Salzsäure nach dem Membranverfahren können Elektroden eingesetzt werden, die Graphit enthalten, wobei die Anode aus Graphit bestehen kann.

In einer bevorzugten Ausführungsform erfolgt die elektrochemische Oxidation der wässrigen Lösung von Chlorwasserstoff gemäß Schritt (3) nach dem Membranverfahren mit einer Gasdiffusionselektrode als Sauerstoffverzehrkathode. Dabei kann die Elektrolysezelle sowohl aus zwei Kammern als auch aus drei Kammern, bevorzugt jedoch aus zwei Kammern, bestehen. Der Kathodenhalbzelle wird ein sauerstoffhaltiges Gas, z.B. Sauerstoff, Luft oder mit Sauerstoff angereicherte Luft, zugeführt. Der Sauerstoff wird an der Gasdiffusionselektrode reduziert, wobei Wasser gebildet wird. Der Anodenhalbzelle wird die wässrige Chlorwasserstoff-Lösung zugeführt, wobei der Chlorwasser-stoff an der Anode zu Chlor oxidiert wird. Die Anodenhalbzelle und die Kathodenhalbzelle sind durch eine Kationenaustauschermembran voneinander getrennt. Die Elektrolyse von Salzsäure unter Verwendung einer Gasdiffusionselektrode als Kathode ist z.B. in WO 00/73538 beschrieben.

Die Elektrolysezelle kann entweder aus einem nichtmetallischen Werkstoff gemäß DE 103 47 703 A oder aus einem metallischen Werkstoff bestehen. Als metallischer Werkstoff für die Elektrolysezelle eignet sich z.B. Titan oder eine Titan-Legierung, wie eine Titan-Palladium-Legierung. Dabei sind die Halbschalen für die Anode- und Kathodenhalbzelle, der Stromverteiler und die Stromzuführungen aus Titan oder einer Titan-Legierung gefertigt.

Die Anode kann z.B. gemäß DE 102 34 806 A ausgeführt sein. Dabei besteht die Anode aus Metall, vorzugsweise aus Titan mit einer Beschichtung aus Edelmetalloxid, z.B. aus Rutheniumoxid. Weiterhin kann die Anode aus Titan gemäß DE 102 00 072 A eine Zwischenschicht aus Titancarbid oder Titanborid aufweisen, welche mittels Plasma- oder Flammspritzen auf die Titananode aufgebracht wird, bevor die Beschichtung aus einem Edelmetalloxid aufgebracht wird. Gemäß DE 102 34 806 A weist die Metallanode Öffnungen für den Durchlass des während der Elektrolyse gebildeten Gases auf, wobei die Öffnungen bevorzugt Leitstrukturen aufweisen, welche das gebildete Gas auf die der Ionenaustauschermembran abgewandten Seite der Metallanode ableiten. Dabei sollte die Gesamtquerschnittsfläche der Öffnungen im Bereich von 20% bis 70% der Fläche betragen, welche durch die Höhe und Breite der Anode gebildet wird. Die Metallanode kann außerdem einen wellenförmigen, zickzackförmigen oder rechteckförmigen Querschnitt aufweisen. Die Tiefe der Anode sollte dabei mindestens 1 mm betragen. Das Verhältnis von elektrochemisch aktiver Fläche der Metallanode zu der Fläche, welche durch die Höhe und Breite der Metallelektrode gebildet wird, sollte mindestens 1,2 betragen. In einer speziellen Ausführungsform kann die Metallanode aus zwei aneinander liegenden Streckmetallen bestehen, wobei das zur Ionenaustauschermembran weisende Streckmetall feiner strukturiert ist als das von der Ionenaustauschermembran abweisende Streckmetall. Dabei ist ferner das feiner strukturierte Streckmetall flach gewalzt und das gröber strukturierte Streckmetall so angeordnet, dass die Maschenstege in Richtung der Kathode geneigt sind und als Leitstrukturen dienen. Alternativ kann die Anode auch aus einem Streckmetall bestehen. Grundsätzlich sollte die Anode eine freie Fläche von 15 bis 70 % aufweisen. Die Dicke der Streckmetalle ist so zu wählen, dass kein zusätzlicher elektrischer Widerstand bei einer bipolaren Verschaltung der einzelnen Elektrolysezellen (Zellelemente) zu einem Elektrolyseur auftritt. Der elektrische Widerstand hängt im Wesentlichen von der elektrischen Kontaktierung der Anode ab, wie zum Beispiel Anzahl der stromzuführenden Verbindungselemente zwischen Anode und Rückwand der Anodenhalbzelle.

Bei der Elektrolyse mittels Gasdiffusionselektrode können der Anodenraum und der Kathodenraum von einer handelsüblichen Ionenaustauschermembran getrennt sein. Beispielsweise können Ionenaustauschermembranen der Fa. DuPont vom Typ Nafion^{®} 324 oder Nafion^{®} 117 eingesetzt werden. Bevorzugt wird eine Membran eingesetzt, welche, wie in WO 05/12596 beschrieben, auf der zur Gasdiffusionselektrode gewandten Seite eine glatte Oberflächenstruktur aufweist. Die glatte Oberflächenstruktur der Membran erlaubt es, dass die Gasdiffusionselektrode und die Membran derart aneinander anliegen, dass unter einem Druck von 250 g/cm² und einer Temperatur von 60 °C die Kontaktfläche wenigstens 50 % der geometrischen Fläche der Membran beträgt.

Der kathodische Stromverteiler, auf den die Gasdiffusionselektrode aufgebracht wird, ist bevorzugt gemäß DE 102 03 689 A ausgeführt. Dieser weist eine freie Fläche von weniger als 65 %, jedoch von mehr als 5 % auf. Die Dicke des Stromverteilers beträgt mindestens 0,3 mm. Er kann aus einem Streckmetall, Netz, Gewebe, Schaum, Vlies, Schlitzblech oder Lochplatte aus Metall bestehen. Vorzugsweise besteht der kathodische Stromverteiler aus einem Streckmetall mit einer Maschenlänge von 4 bis 8 mm, einer Maschenbreite 3 bis 5 mm, einer Stegbreite von 0,4 bis 1,8 mm und einer Dicke von 0,4 bis 2 mm. Zusätzlich kann der kathodische Stromverteiler ein zweites Streckmetall als Träger für das erste Streckmetall aufweisen. Das zweite Streckmetall als Träger hat bevorzugt eine Maschenlänge von 10 bis 40 mm, eine Maschenbreite von 5 bis 15 mm, eine Stegbreite von 2 bis 5 mm sowie eine Dicke von 0,8 bis 4 mm. Als Träger kann auch ein Netz eingesetzt werden, welches vorzugsweise eine Drahtdicke von 1 bis 4 mm und eine Maschenweite von 7 bis 25 mm besitzt. Weiterhin kann als Träger ein Lochblech oder Schlitzblech eingesetzt werden, welches bevorzugt eine offene Fläche von weniger als 60 % und eine Dicke von 1 bis 4 mm besitzt. Als Werkstoff für den kathodischen Stromverteiler kann z.B. Titan oder eine edelmetallhaltige Titanlegierung, wie z.B. Titan-Palladium eingesetzt werden. Ist der Stromverteiler ein Streckmetall, so ist dieses bevorzugt gewalzt.

Als Gasdiffusionselektrode kann eine handelsübliche Gasdiffusionselektrode eingesetzt werden, die mit einem geeigneten Katalysator ausgerüstet ist. Geeignete Katalysatoren enthalten gemäß WO 00/73538 Rhodium und/oder wenigstens ein Rhodiumsulfid oder eine Mischung aus Rhodium und wenigstens einem Rhodiumsulfid. Gemäß EP 931 857 A können außerdem Rhodium und/oder Rhodiumoxid oder deren Mischungen eingesetzt werden. Die Gasdiffusionselektrode besteht bevorzugt aus einem elektrisch leitfähigen Gewebe, Papier oder Vlies aus Kohlenstoff, wobei das Gewebe, Papier oder Vlies auf einer Seite mit einer kohlenstoffhaltigen Katalysatorschicht und auf der anderen Seite mit einer Gasdiffusionsschicht versehen ist. Der Katalysator wird bevorzugt auf einen Träger, vorzugsweise aus Kohlenstoff, aufgebracht, wobei Polytetrafluorethylen-Teilchen integriert sind, die an die Trägerstruktur gekoppelt sind. Die Gasdiffusionsschicht besteht vorzugsweise aus Kohlenstoff und Polytetrafluorethylen-Teilchen, bei der beispielsweise das Verhältnis von Kohlenstoff zu PTFE 50:50 beträgt. Die Gasdiffusionselektrode kann beispielsweise so angeordnet werden, dass sie nicht fest mit der Ionenaustauschermembran verbunden ist. Die Kontaktierung der Gasdiffusionselektrode mit dem Stromverteiler und der Ionenaustauschermembran erfolgt bevorzugt durch Presskontakt, d.h. die Gasdiffusionselektrode, der Stromverteiler und die Membran werden aneinander gepresst. Die Gasdiffusionselektrode kann mit dem Stromkollektor gemäß DE 101 48 600 A verbunden sein.

Die Elektrolyse von Salzsäure nach dem Membranverfahren mit Gasdiffusionselektrode wird üblicherweise bei einer Temperatur von 40 bis 70°C durchgeführt. Die Konzentration der wässrigen Lösung von Chlorwasserstoff im Anodenraum beträgt 10 bis 20 Gew.%, bevorzugt 12 bis 17 Gew.%. Die Zelle kann beispielsweise so betrieben werden, dass der Druck im Anodenraum höher ist als der Druck im Kathodenraum. Dadurch wird die Kationenaustauschermembran auf die Gasdiffusionselektrode und diese wiederum auf den Stromverteiler gedrückt. Alternativ kann eine Konstruktion der Elektrolysezelle gewählt werden, die in DE 101 38 214 A beschrieben wird. Die Anode und/oder der Stromverteiler sind elastisch gelagert, beispielsweise indem sie mittels Federn mit der Rückwand der jeweiligen Halbzelle verbunden sind. Beim Zusammenbau der Zelle entsteht eine sogenannte zero gap Anordnung, bei der die Anode in direktem Kontakt mit der Ionenaustauschermembran, diese wiederum in direktem Kontakt mit der Gasdiffusionselektrode und diese wiederum in direktem Kontakt mit dem Stromverteiler steht. Die elastische Lagerung bewirkt das Aneinanderpressen von Anode, Membran, Gasdiffusionselektrode und Stromverteiler.

In einer bevorzugten Ausführungsform des Elektrolyseverfahrens wird beim Anfahren der Elektrolysezelle gemäß DE 10 152 275 A das Anodenhalbelement wird mit einer 5 bis 20 Gew.%-igen Salzsäure gefüllt, wobei die Salzsäure mindestens 10 ppm freies Chlor enthält und die Konzentration der Salzsäure während der Inbetriebnahme mehr als 5 Gew.-% beträgt. Der Volumenstrom der Salzsäure durch den Anodenraum wird so eingestellt, dass zu Beginn der Elektrolyse die Salzsäure mit einer Geschwindigkeit im Anodenraum von 0,05 bis 0,15 cm/s strömt. Die Elektrolyse wird mit einer Stromdichte von 0,5 bis 2 kA/m² gestartet und in Zeitintervallen von 5 bis 25 Minuten um jeweils 0,5 bis 1,5 kA/m² erhöht. Nachdem eine vorgegebene Stromdichte von vorzugsweise 4 bis 7 kA/m² erreicht ist, wird der Volumenstrom der Salzsäure so eingestellt, dass die Salzsäure im Anodenhalbelement mit einer Geschwindigkeit von 0,2 bis 0,4 cm/s strömt.

Eine besonders vorteilhafte Betriebsweise der Elektrolysezelle kann gemäß DE 101 38 215 A erfolgen, wonach die Elektrolysezelle zur Erniedrigung der Zellspannung mit einem erhöhten Druck in der Kathodenkammer betrieben wird. Der Differenzdruck zwischen Anodenraum und Kathodenraum sollte 0,01 bis 1000 mbar und der Sauerstoffdruck im Kathodenraum mindestens 1,05 bar absolut betragen.

Die Kreislauffahrweise des erfindungsgemäßen Verfahrens erfordert es, neben dem gemäß Schritt (e) mittels thermische Oxidation hergestellten Chlors für die Phosgenherstellung gemäß Schritt (a) eine weitere Teilmenge Chlor bereitzustellen, da Verluste an Chlor und Chlorwasserstoff in dem Chlor-Chlorwasserstoff-Kreislauf auftreten. Die Bereitstellung einer weiteren Teilmenge Chlor kann in Form von elementarem Chlor aus einer externen Quelle, beispielsweise der Elektrolyse einer wässrigen Natriumchlorid- oder Salzäurelösung, stammen. Die auftretenden Verluste von Chlor und Chlorwasserstoff können aber auch dadurch ausgeglichen werden, dass eine Teilmenge Chlorwasserstoff aus einer externen Quelle bereitgestellt wird, z.B. aus einer Isocyanatproduktionsanlage (e.g. MDI, TDI), bei dem Chlorwasserstoff als gasförmiges Nebenprodukt anfällt. Eine Teilmenge Chlorwasserstoff in Form einer wässrigen Chlorwasserstofflösung aus einer externen Quelle, z.B. aus einem Produktionsverfahren, bei dem eine wässrige Chlorwasserstofflösung als Nebenprodukt anfällt, wird vorzugsweise als ca 30 Gew.%-ige Salzsäure einer Elektrolyse zugeführt. Eine Salzsäure geringerer Konzentration kann alternativ der Absorption von Chlorwasserstoff zugeführt werden.

Das Phosgen kann wieder in die Herstellung des Diarylcarbonats gemäß Schritt (b) eingesetzt werden.

Die Umesterung des gemäß Schritt (b) hergestellten Diarylcarbonats mit wenigstens einem Bisphenol ergibt ein Oligo-/Polycarbonat und Monophenol, das wiederum in Schritt (b) eingesetzt werden kann.

Für das erfindungsgernäße Verfahren geeignete Bisphenole sind Dihydroxydiarylalkane der Formel (II),

HO-Z-OH (II)

worin Z ein divalenter organischer Rest mit 6 bis 30 Kohlenstoffatomen ist, der eine oder mehrere aromatische Gruppen enthält. Beispiele solcher Verbindungen, die in dem erfindungsgemäßen Verfahren eingesetzt werden können sind Dihydroxydiarylalkane wie Hydrochinon, Re-sorcin, Dihydroxydiphenyl, Bis-(hydroxyphenyl)-alkane, Bis(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, a,a'-Bis-(hydroxyphenyl)-diisopropylbenzole, so wie deren alkylierte, kernalkylierte und kernhalogenierte Verbindungen.

Bevorzugte Bisphenole sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)-propan (Bisphenol A (BPA)), 2,4-Bis-(4-hydroxyphenyl)-2-methylbutan, 1,3-Bis-[2-(4-hydroxyphenyl)-2-propyl]-benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1,1-Bis-(4-hydroxyphenyl)-cyclohexan und 1,1-Bis-(4-hydroxyphenyl)-3,3,5-trimethyl-cyclohexan (Bisphenol TMC).

Besonders bevorzugte Bisphenole sind 4,4'-Dihydroxydiphenyl, 1,1-Bis-(4-Hydroxyphenyl)-phenylethan, 2,2-Bis-(4-hydroxyphenyl)-propan (Bisphenol A (BPA)), 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Geeignete Bisphenole sind z.B. in den US 2 999 835 A, US 3 148 172 A, US 2 991 273 A, US 3 271 367 A, US 4 982 014 A und US 2 999 846 A in den deutschen Offenlegungsschriften DE 15 70 703 A, DE 20 63 050 A, DE 20 36 052 A, DE 22 11 956 A und DE 38 32 396 A, der französischen Patentschrift FR 1 561 518 A, in der Monographie von H. Schnell, Chemistry and Physics of Polycarbonates, Interscience Publishers, New York 1964, S. 28ff; S.102ff und von D.G. Legrand, J.T. Bendler, Handbook of Polycarbonate Science and Technology, Marcel Dekker New York 2000, S. 72ff. beschrieben.

Im Falle der erfindungsgemäßen Herstellung von Homopolycarbonaten wird nur ein Bisphenol eingesetzt, im Falle der erfindungsgemäßen Herstellung von Copolycarbonaten werden mehrere Bisphenole eingesetzt, wobei selbstverständlich die verwendeten Bisphenole, wie auch alle anderen der Synthese zugesetzten Chemikalien und Hilfsstoffe mit den aus ihrer eigenen Synthese, Handhabung und Lagerung stammenden Verunreinigungen kontaminiert sein können, obwohl es wünschenswert ist, mit möglichst sauberen Rohstoffen zu arbeiten.

Es sei hier betont, dass das erfindungsgemäße Verfahren praktisch für alle bekannten Bisphenole eingesetzt werden kann.

Die Polycarbonate können durch den Einsatz geringer Mengen Kettenabbrecher und Verzweiger bewusst und kontrolliert modifiziert werden. Geeignete Kettenabbrecher und Verzweiger sind literaturbekannt. Einige sind beispielsweise in der DE-A 38 33 953 beschrieben. Bevorzugt eingesetzte Kettenabbrecher sind Phenol oder Alkylphenole, insbesondere Phenol, p-tert.Butylphenol, iso-Octylphenol, Cumylphenol, deren Chlorkohlensäureester oder Säurechloride von Monocarbonsäuren bzw. Gemischen aus diesen Kettenabbrechern. Bevorzugte Kettenabbrecher sind Phenol, Cumyl-phenol, Isooctylphenol und para-tert.-Butylphenol.

Beispiele für als Verzweiger geeignete Verbindungen sind aromatische oder aliphatische Verbindungen mit mehr als drei, bevorzugt drei oder vier Hydroxygruppen. Besonders geeignete Beispiele mit drei oder mehr als drei phenolischen Hydroxylgruppen sind Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-(4,4-bis-(4-hydroxyphenyl)-cyclohexyl]-propan, 2,4-Bis-(4-hydroxyphenyl-isopropyl)-phenol und Tetra-(4-hydroxyphenyl)-methan.

Beispiele für sonstige als Verzweiger geeignete trifunktionelle Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Besonders bevorzugte Verzweiger sind 3,3-Bis-(3-methyl-4-hydroxyphenyl)-2-oxo-2,3-dihydro-indol und 1,1,1-Tri-(4-hydroxyphenyl)-ethan.

Die gegebenenfalls mitzuverwendenden 0,05 bis 2 Mol-%, bezogen auf eingesetzte Bisphenole, an Verzweigern, können mit den Bisphenolen zusammen eingesetzt werden.

Es ist darauf zu achten, dass die Reaktionskomponenten für den ersten Schritt, die Umesterung, also, die Bisphenole und die Diarylcarbonate frei von Alkali und Erdalkaliionen sind, wobei Mengen von kleiner 0,1 ppm an Alkali- und Erdalkaliionen toleriert werden können. Derart reine Bisphenole bzw. Diarylcarbonate sind erhältlich, indem man die Bisphenole bzw. Diarylcarbonate umkristallisiert, wäscht oder destilliert. Beim erfindungsgemäßen Verfahren soll der Gehalt an Alkali- und Erdalka-limetallionen sowohl im Bisphenol als auch im Diarylcarbonate einen Wert von < 0,1 ppm betragen.

Die Umesterungsreaktion des Bisphenols und des Diarylcarbonats in der Schmelze wird bevorzugt in zwei Stufen durchgeführt. In der ersten Stufe findet das Aufschmelzen des Bisphenols und des Diarylcarbonats bei Temperaturen von 80 - 250°C, bevorzugt 100 - 230°C, besonders bevorzugt 120 -190°C unter normalem Druck in 0-5 Stunden, bevorzugt 0,25 - 3 Stunden statt. Nach Zugabe des Katalysators wird durch Anlegen von Vakuum (bis zu 2 mm Hg) und Erhöhung der Temperatur (auf bis zu 260°C) durch Abdestillieren des Monophenols das Oligocarbonat aus dem Bisphenol und dem Diarylcarbonat hergestellt. Das so hergestellte Oligocarbonat hat eine mittlere Gewichtsmol-masse M_{w} (ermittelt durch Messung der rel. Lösungsviskosität in Dichlormethan oder in Mischungen gleicher Gewichtsmengen Phenol / o-Dichlorbenzol, geeicht durch Lichtstreuung) im Bereich von 2000 bis 18 000 bevorzugt von 4 000 bis 15 000. Hierbei wird die Hauptmenge an Monophenol (80 %) aus dem Prozeß wiedergewonnen.

In der zweiten Stufe wird bei der Polykondensation durch weiteres Erhöhen der Temperatur auf 250 - 320°C, bevorzugt 270 - 295°C und einem Druck von < 2 mm Hg das Polycarbonat hergestellt. Hierbei wird der Rest an Monophenolen zurückgewonnen. Es können geringe Verluste an Monophenolen < 5 %, bevorzugt < 2 %, besonders bevorzugt < 1 % auftreten, verursacht durch Endgruppen im Polycarbonat und Restmonophenol im Polycarbonat. Diese Verluste müssen durch entsprechende Mengen an Monophenol für die Herstellung des Diarylcarbonats ausgeglichen werden.

Katalysatoren im Sinne des erfindungsgemäßen Verfahren für die Umesterung des Bisphenols und des Diarylcarbonats zum Polycarbonat sind alle anorganische oder organischen basischen Verbindungen beispielsweise Lithium-, Natrium-, Kalium-, Cäsium-, Calzium-, Barium-, Magnesium-, - hydroxide, -carbonate, -halogenide, -phenolate, -diphenolate, -fluoride, -acetate, -phosphate, - hydrogenphosphate, -boranate, Stickstoff- und Phosphorbasen wie beispielsweise Tetramethylammoniumhydroxid, Tetramethylammoniumacetat, Tetramethylammoniumfluorid, Tetramethylammoniumtetraphenylboranat, Tetraphenylphosphoniumfluorid, Tetraphenylphosphoniumtetraphenylboranat, Dimethyldiphenylammoniumhydoxid, Tetraethylammoniumhydroxid, DBU, DBN oder Guanidinsysteme wie beispielsweise das 1,5,7-Triazabicyclo-[4,4,0]-dec-5-en, 7-Phenyl-1,5,7-triazabicyclo-[4,4,0]-dec-5-en, 7-Methyl-1,5,7-triazabicyclo-[4,4,0]-dec-5-en, 7,7'-Hexyliden-di-1,5,7-triazabicyclo-[4,4,0]-dec-5-en, 7,7'-Decyliden-di-1,5,7-triazabicyclo-[4,4,0]-dec-5-en, 7,7'-Dodecyliden-di-1,5,7-triazabicyclo-[4,4,0] -dec-5-en oder Phosphazene wie beispielsweise die Phosphazen-Base P₁-t-Oct = tert.-Octyl-imino-tris-(dimethylamino)-phosphoran, Phosphazen-Base P₁-t-Butyl = tert.-Butyl-imino-tris-(dimethylamino)-phosphoran, BEMP = 2-tert.-Butylimino-2-diethylamino-1,3-dimethylperhydro-1,3,2-diaza-2-phosphoran.

Diese Katalysatoren werden in Mengen von 10⁻² bis 10⁻⁸ Mol, bezogen auf 1 Mol Bisphenol, eingesetzt.

Die Katalysatoren können auch in Kombination (zwei oder mehrere) miteinander eingesetzt werden.

Beim Einsatz von Alkali-/Erdalkali-Metallkatalysatoren kann es vorteilhaft sein, die Alkali-/ Erdalkali-Metallkatalysatoren zu einem späteren Zeitpunkt (z.B. nach der Oligocarbonatsynthese bei der Polykondensation in der zweiten Stufe) zuzusetzen. Die Zugabe des Alkali-/Erdalkali-Metallkatalysators kann z.B. als Feststoff oder als Lösung in Wasser, Phenol, Oligocarbonat oder Polycarbonat erfolgen.

Die Mitverwendung von Alkali- bzw. Erdalkali-Metallkatalysatoren widerspricht nicht der vorstehend erwähnten Forderung nach Reinheit der Reaktionspartner.

Die Reaktion des Bisphenols und des Diarylcarbonats zum Polycarbonat kann im Sinne des erfindungsgemäßen Verfahrens diskontinuierlich oder bevorzugt kontinuierlich durchgeführt werden, beispielsweise in Rührkesseln, Dünnschichtverdampfern, Fallfilmverdampfern, Rührkesselkaskaden, Extrudern, Knetern, einfachen Scheibenreaktoren und Hochviskosscheibenreaktoren.

Die aromatischen Polycarbonate des erfindungsgemaßen Verfahrens sollen mittlere Gewichtsmolmassen M_{w} von 18000 bis 80000 vorzugsweise 19000 bis 50000 haben, ermittelt durch Messung der rel. Lösungsviskosität in Dichlormethan oder in Mischungen gleicher Gewichtsmengen Phenol / o-Dichlorbenzol, geeicht durch Lichtstreuung.

Es ist vorteilhaft, die aus der Umesterung des Bisphenols und des Diarylcarbonats zum Polycarbonat abgespaltenen und isolierten Monophenole vor dem Einsatz in die Diarylcarbonatsynthese aufzureinigen. Die Rohmonophenole, die beim Umesterungsprozeß isoliert werden, können je nach Umesterungsbedingungen und Destillationsbedingungen u.a. mit Diarylcarbonaten, dem Bisphenol, Salicylsäure, Isopropenylphenol, Phenoxybenzoesäurephenylester, Xanthon, dem Hydroxymonoarylcarbonat verunreinigt sein. Die Reinigung kann nach den üblichen Reinigungs-verfahren, also z. B. Destillation oder Umkristallisation erfolgen. Die Reinheit der Monophenole liegt dann bei > 99 %, bevorzugt bei > 99,8 %, besonders bevorzugt bei > 99,95 %.

Die Vorteile des erfindungsgemäßen integrierten Verfahrens zur Herstellung von Diarylcarbonaten und Polycarbonaten unter thermischer Oxidation des bei der Diarylcarbonatherstellung anfallenden Chlorwasserstoffs mit einem sauerstoffhaltigen Gas nach dem Deacon-Verfahren zur Rückgewinnung von Chlor für die Synthese von Phosgen sind der wirtschaftlichere Betrieb unter Vermeidung der Wasserstoffproduktion in Vergleich mit einer klassischen Elektrolyse.

Die Herstellung von Diarylcarbonaten und Polycarbonaten im Kombination mit der thermischen Oxidation von Chlorwasserstoff bietet durch die Erzeugung einer aufkonzentrierten Salzsäure von rund 30% aus Chlorwasserstoff zusätzlich die Möglichkeit, bei Bedarf aufkonzentrierte Salzsäure für andere Anwendungen dem Kreislauf zu entnehmen. Eine mögliche Verwendung dieser aufkonzentrierten Salzsäure liegt im Nahrungsmittelbereich. Hierfür kann für die nach dem erfindungsgemäßen Verfahren hergestellte aufkonzentrierte Salzsäure z.B. durch absorptive Nachreinigung an einem Aktivkohlebett, wie sie aus dem Stand der Technik bekannt ist, eine für die Nahrungsmittelindustrie ausreichend hohe Reinheit erreicht werden.

Das erfindungsgemäße Verfahrens zur Herstellung von Diarylcarbonaten und Polycarbonaten kann gegebenenfalls mit anderen Einsatzmöglichkeiten des bei der Diarylcarbonatherstellung anfallenden Chlorwasserstoffs wie z. B. die Verwendung als Edukt für die Ethylendichloridherstellung bei der PVC-Produktion kombiniert werden.

Das nach der thermischen Oxidation von Chlorwasserstoff in Gegenwart eines Katalysators nach dem Deacon-Verfahren noch Chlorwasserstoff enthaltende Prozessgas wird einer Chlorwasserstoffabsorptionsanlage zugeführt, wobei neben gereinigter Chlorwasserstoff aus mehreren Quellen, abhängig vom nachfolgenden Oxidationsverfahren eine an Salzsäure abgereicherten Anolytsäure aus einer Chlorwasserstoff-Elektrolyse oder eine an Natriumchlorid verarmten Natriumchloridlösung aus einer Chloralkali-Elektrolyse eingespeist werden kann.
**Fig. 1** zeigt ein integriertes Verfahren zur Herstellung von Polycarbonat und Rückführung des anfallenden Chlorwasserstoffs und des Phenols.
**Fig. 2** zeigt ein integriertes Verfahren zur Herstellung von Diarylcarbonat (Diphenylcarbonat, DPC) und Rückführung des anfallenden Chlorwasserstoffs
**Fig. 3** zeigt ein Verfahren zur Herstellung von Diphenylcarbonat durch Direktphosgenierung von Phenol in Gegenwart von heterogenen Katalysatoren

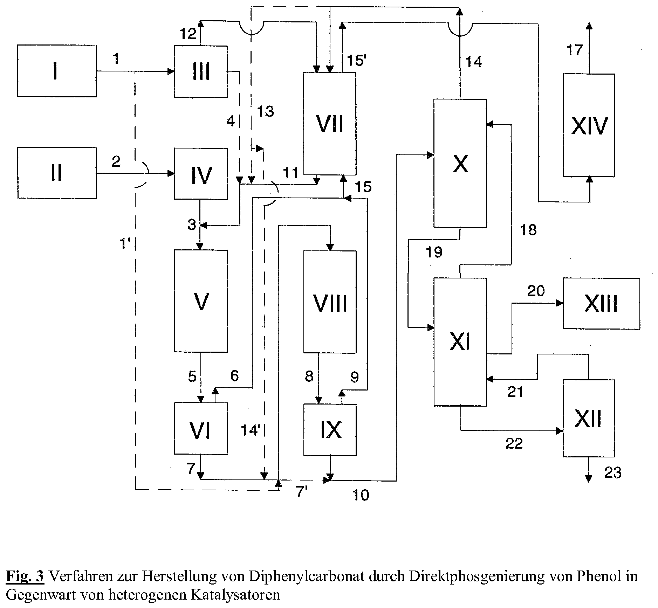

Folgende Bezugszeichen wurden in Fig. 3 verwendet:
- I: Vorratsbehälter Phenol
- II: Vorratsbehälter Phosgen
- III, IV: Wärmetauscher
- V, VIII: Reaktor
- VI; IX: Entgaser
- VII: Gegenstromapparatur
- X, XI, XII: Destillationskolonne
- XIII: Produktbehälter
- XIV: Reinigungsanlage

### Beispiele

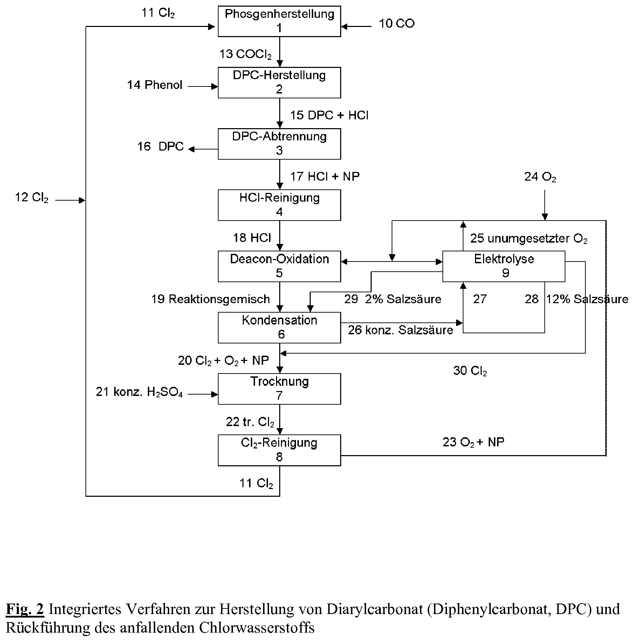

Die Beispiele sollen das erfindungsgemäße Verfahren anhand der thermischen Oxidation der bei der Herstellung von Diphenylcarbonat anfallenden Chlorwasserstoff zu Chlor, dessen Umsetzung zu Phosgen, das wieder in das Diphenylcarbonat-Herstellungsverfahren (DPC-Verfahren) eingesetzt werden kann (Figur 2), unter Rückführung des bei der Herstellung von Polycarbonat gebildeten Phenols in die Herstellung von Diphenylcarbonat (Figur 1), darstellen.

In einer ersten Stufe 1 der Diarylcarbonatherstellung (Figur 2) wird Chlor 11 mit Kohlenmonoxid 10 zu Phosgen 13 umgesetzt. In der folgenden Stufe 2 wird Phosgen 13 aus Stufe 1 mit einem Monophenol 14 (z.B. Phenol) zu einem Gemisch 15 aus Diarylcarbonat (z.B. Diphenylcarbonat, DPC) und Chlorwasserstoff umgesetzt, das in Stufe 3 in Diarylcarbonat 16, das verwertet wird, und HCl-Gas 17, das einer Reinigung 4 unterzogen wird, aufgetrennt wird. Das gereinigte HCl-Gas 18 wird in dem HCl-Oxidationsverfahren 5 mit Sauerstoff umgesetzt, hier in einem Deacon-Prozess mittels Katalysator.

Das Reaktionsgemisch 19 aus Stufe 5 wird abgekühlt (Stufe 6). Nicht umgesetzte Chlorwasserstoff 26, die dabei, gegebenenfalls mit Wasser oder verdünnter Salzsäure vermischt anfällt, wird ausgeschleust und einer Elektrolyse 9 zugeführt.

Das so erhaltene Gasgemisch 20 bestehend wenigstens aus Chlor, Sauer-stoff und Nebenbestandteilen wie Stickstoff, Kohlendioxid usw. wird mit konz. Schwefelsäure 21 (96 %-ig) getrocknet (Stufe 7).

In einer Reinigungsstufe 8 wird Chlor 11 vom Gasgemisch 22 aus Stufe 7 getrennt. Der Sauerstoff und Nebenbestandteile aufweisende Reststrom 23 wird in Stufe 5 (Deacon-Oxidation) und Stufe 9 (Elektrolyse) rückgeführt. Die Elektrolysestufe ist eine ODC-Elektrolyse in der auf der Kathodenseite eine Gasdiffusionselektrode und Sauerstoff als Reaktand eingesetzt wird.

Das aus der Reinigungsstufe 8 erhaltene Chlorgas 11 kann direkt in der Phosgensynthese 1 wieder eingesetzt werden.

Die Konzentration der der Elektrolysezelle 9 zugeführten Salzsäure 27 beträgt 14 bis 15 Gew.- % HCl, die der aus der Elektrolysezelle 9 ablaufenden Salzsäure 28 beträgt 11 bis 13 Gew.- % HCl. Der Salzsäure-Strom 28 wird mit konzentrierter Salzsäure 26 aus der Trennungsstufe 6 versetzt und erneut der Elektrolysezelle 9 zugeführt.

Der bei den Stufen 5 und 9 verbrauchte Sauerstoff wird durch Sauerstoff aus einer externen Quelle 24 ersetzt. Der im Kathodenraum der Elektrolysezelle nicht verbrauchte Sauerstoff 25 wird im Kreis geführt und mit frischem Sauerstoff aus einer externen Quelle 24 versetzt.
Der ebenfalls im Kathodenraum anfallende ca. 2 Gew.%-ige Salzsäurestrom 29 wird der Salzsäure-Abtrennung 6 zugeführt und dient dort als Absorptionsmittel für überschüssigen gasförmige Chlorwasserstoff.

Das gemäß Stufe 9 hergestellte Chlor 30 wird mit dem chlorhaltigen Gasstrom 20 vereinigt und der vereinigte Gasstrom in einer ein- oder mehrstufigen Kühlung mittels eines Kühlaggregats, z.B. eines Röhrenwärmetauscher, abgekühlt und getrocknet.

Die Trocknung 7 kann zum Beispiel mit Hilfe eines geeigneten Trocknungsmittels in einer mit Stoffaustauschelementen bestückten Absorptionskolonne erfolgen. Ein geeignetes Trocknungsmittel kann, wie z.B. in DE 10 235 476 A beschrieben, neben Molsieben oder hygroskopischen Adsorbentien z.B. Schwefelsäure sein. Die Trocknung kann ein- oder mehrstufig erfolgen. Bevorzugt erfolgt die Trocknung zweistufig, indem das zu trocknende Chlor in einer ersten Stufe mit einer Schwefelsäure geringe-rer Konzentration, vorzugsweise 70 bis 80 %, besonders bevorzugt 75 bis 80 %, in Kontakt gebracht wird. In einer zweiten Stufe wird die Restfeuchte mittels einer höher konzentrierten Schwefelsäure von bevorzugt 88 bis 96 %, besonders bevorzugt 92-96 %, aus dem Chlor entfernt. Das auf diese Art getrocknete Chlor 22 mit einer Restfeuchte von bevorzugt maximal 100 ppm, besonders bevorzugt maximal 20 ppm kann durch einen Tröpfchenabscheider geleitet werden, um gegebenenfalls darin noch enthaltene Schwefelsäuretröpfchen zu entfernen.

Der getrocknete Chlorgasstrom 22 wird anschließend einer Chlorreinigung 8 unterzogen.

Das sehr reine Chlor 11 wird mit Chlor aus einer externe Quelle 12 ergänzt und wieder der Phosgenherstellung 1 zugeführt.

### Beispiele 1a-h

### Herstellung von Diphenylcarbonat durch Direktphosgenierung von Phenol

### 1a) Diphenylcarbonat durch Phosgenierung von Phenol in Gegenwart von γ-Al₂O₃ (Referenzbeispiel)

Die zur Durchführung des erfindungsgemäßen Verfahrens eingesetzte Apparatur und die auftretenden Stoffströme sind schematisch in Figur 3 wiedergegeben.

Aus einem beheizten Behälter I dosiert man über Wärmetauscher III (60°C) unter Normaldruck 41,12 Gew.-Teile/h Phenol 1, frisch und/oder zurückgewonnen aus der Polycarbonatherstellung, wie in Bsp 3) beschrieben, von oben in eine auf 60°C beheizte mit Füllkörpern beschickte Gegenstromkolonne VII, in welcher eine Vermischung mit aus der Destillationskolonne X am Kopf entnommenen Phenol 14 stattfindet. Nach Durchströmen des aus den Entgasungsapparaturen VI und IX stammenden Abgasstroms 15 durch VII wird Mischung 11, (Gewichtsverhältnis Phenol/Phosgen ca. 97/3) am Fuß ausgespeist. Aus dem Vorratsbehälter II werden 21,93 Gew.-Teile/h über Wärmetauscher IV (170°C) vorgeheiztes Phosgen 3 zusammen mit 11 in einem auf 170°C beheizten, mit 150 Vol.-Teilen γ-Aluminiumoxid gefüllten Reaktor V in Gleichstrom eingeleitet.

Das am Fuß des Reaktors austretende Produkt 5, das Phenol, Chlorameisensäurephenylester, Diphenylcarbonat und Nebenprodukte im Verhältnis 56,1/0,8/ 42,8/0,3 enthält, wird über Entgaser VI in Abgas 6 (Gewichtsverhältnis Phenol, Phosgen, Chlorwasserstoff und Kohlendioxid 2,5/15,8/81,1/0,6) und Sumpf 7 (Gewichtsverhältnis Phenol, Chlorameisensäurephenylester, Diphenylcarbonat und Nebenprodukte 55,8/0,9/43,0/0,3) aufgetrennt.

Der im Sumpf 7 vorhandene Chlorameisensäurephenylester wird durch Nachreaktion mit vorhandenem Phenol (eventuell nach Zugabe von zusätzlichem Phenol (1' oder 14') in einem Nachreaktor VIII, ebenfalls befüllt mit γ-Aluminiumoxid (150 Vol.-Teilen) bei 180°C zu Diphenylcarbonat umgesetzt.

Das am Reaktorfuß entnommene Produkt 8 (Gewichtsverhältnis Phenol, Diphenylcarbonat und Nebenprodukte 55,4/44,3/0,3) wird über Entgaser IX in Abgas 9 (Phenol/Chlorwasserstoff) und Sumpf 10 (Gewichtsverhältnis Phenol, Diphenylcarbonat und Nebenprodukte 55,2/44,5/0,3) aufgetrennt.

Sumpf 10 wird in eine erste Destillationskolonne X eingespeist und bei ca. 80°C/16 mbar (12 mm) aufgetrennt in 57,7 Gew.-Teile/h Phenol und Sumpf 19 (Gewichtsverhältnis Phenol, Diphenylcarbonat und Nebenprodukte 0,3/99,1/0,6). Sumpf 19 wird einer zweiten Destillationskolonne XI zugeleitet, in der über Kopf noch vorhandenes Phenol (0,14 Gew.-Teile) entfernt und in den oberen Teil der ersten Kolonne X zurückgegeben wird. Das am Fuß entnommene Produkt 22 (Gewichtsverhältnis Diphenylcarbonat/Nebenprodukte 88,6/11,4) wird in einer dritten Destillationskolonne XII bei 170°C/12mm in Kopfprodukt 21 (2,3 Gew.-Teile/h Diphenylcarbonat), das in den unteren Teil der zweiten Kolonne XI zurückgeführt wird, und Sumpf 23 (hochsiedende Nebenprodukte) getrennt. Durch seitliche Ausspeisung aus dem Gasraum der 2. Kolonne XI erhält man 46,6 Gew.-Teile/h Produkt 20 (Gewichtsverhältnis Diphenylcarbonat, Phenol 99,8/0,2), das Produktbehälter XIII zugeführt wird.

Abgasströme 6 und 9 werden vereinigt zu 15 (Gewichtsverhältnis Phenol, Chlorwasserstoff und Kohlendioxid 5,8/93,6/0,6) und in der Gegenstromapparatur VII durch Phenol geleitet. Das am Kopf austretende Abgas 15' wird einer Reinigungsanlage XIV zugeführt, wo das Chlorwasserstoffgas durch Ausfrieren von Phenol befreit wird.

### 1b) Phosgenierung von Phenol in Gegenwart von TiCl₄ in einer Reaktivdestillationskolonne

Man dosiert von oben in eine auf 160°C thermostatisierte Reaktivdestillationskolonne mit 10 Böden von je 18 Volumeteilen unter Normaldruck 49,9 Gew.-Teile/h aufgeschmolzenes Phenol und 0,25 Gew.-Teile/h TiCl₄ und gleichzeitig von unten 25,1 Gew.-Teile/h auf gleicher Temperatur erhitztes Phosgen in Gegenstrom.

Das am Fuß austretende Produkt, enthaltend Chlorameisensäurephenylester und Diphenylcarbonat im Gewichtsverhältnis 3 zu 97, (Phenolumsatz 69,5%, Selektivität 99,6%) wird nach Umsetzung des vorhandenen Chlorameisensäurephenylesters mit Phenol zu Diphenylcarbonat in einem Verweilzeitreaktor isoliert und durch Entgasung in Abgas und Sumpf aufgetrennt.

Der am Kopf der Reaktivdestillationsanlage austretende Abgasstrom wird mit dem Abgas aus der Entgasung vereinigt und von geringen Mengen Phosgen und Phenol durch Ausfrieren befreit.

Der Phosgenumsatz wurde gemessen durch Bestimmung des Phosgengehalts in einem im Abgasstrom eingebauten Gasmaus durch Reaktion mit Überschuß Ethanol und Korrelation des gebildeten Chlorameisensäure ethyl ester und Diethylcarbonat mit der Menge nicht abreagiertes Phosgen.

### 1c) Phosgenierung von Phenol in Gegenwart von TiCl₄ in einer Reaktivdestillationskolonne

Man dosiert von oben in eine auf 150°C thermostatisierte Reaktivdestillationsapparatur mit 20 Böden von je 43 Volumeteilen unter Normaldruck 187,3 Gew.-Teile/h aufgeschmolzenes Phenol und 0,42 Gew.-Teile/h TiCl₄ und gleichzeitig von unten 62,7 Gew.-Teile/h auf gleicher Temperatur erhitztes Phosgen in Gegenstrom.

Das am Fuß austretende Produkt, enthaltend Phenol, Chlorameisensäurephenylester, Diphenylcarbonat und Nebenprodukte (Gewichtsverhältnis 31,7/5,2/62,8/0,3) (Phosgenumsatz 83,5%, Phenolumsatz 64,8%, Selektivität 99,7%) wird nach Umsetzung des vorhandenen Chlorameisensäurephenylesters mit Phenol zu Diphenylcarbonat in einem Verweilzeitreaktor isoliert und durch Entgasung in Abgas und Sumpf (Gewichtsverhältnis Phenol, Diphenylcarbonat und Nebenprodukte 29,2/70,4/0,4) aufgetrennt.

Nach Destillation erhält man 99,8%-iges Diphenylcarbonat.

Der am Kopf der Reaktivdestillationsanlage austretende Abgasstrom wird mit dem Abgas aus der Entgasung vereinigt und von geringen Mengen Phosgen und Phenol durch Ausfrieren befreit.

### 1d) Phosgenierung von Phenol in Gegenwart von AlCl₃ in einer Reaktivdestillationskolonne

Man dosiert von oben in eine auf 150°C thermostatisierte Reaktivdestillationsapparatur mit 20 Böden von je 43 Volumeteilen unter Normaldruck 399,0 Gew.-Teile/h aufgeschmolzenes Phenol und 0,61 Gew.-Teile/h AlCl₃ und gleichzeitig von unten 100,0 Gew.-Teile/h auf gleicher Temperatur erhitztes Phosgen in Gegenstrom.

Das am Fuß austretende Produkt, enthaltend Phenol, Chlorameisensäure phenylester, Diphenylcarbonat und Nebenprodukte (Gewichtsverhältnis 60,7/3,5/34,8/0,6) (Phosgenumsatz 71,6%, Phenolumsatz 35,4%, Selektivität 99%) wird nach Umsetzung des vorhandenen Chlorameisensäurephenylesters mit Phenol zu Diphenylcarbonat in einem Verweilzeitreaktor isoliert und durch Entgasung in Abgas und Sumpf (Gewichtsverhältnis Phenol, Diphenylcarbonat und Nebenprodukte 59,6/39,6/0,8) aufgetrennt.

Das Abgas wird mit dem am Kopf der Reaktivdestillationsanlage austretende Abgasstrom vereinigt und von geringen Mengen Phosgen und Phenol durch Ausfrieren befreit.

Nach Destillation bei 172°C/22 mbar wird 99,7%-iges Diphenylcarbonat erhalten.

### 1e) Phosgenierung von Phenol in Gegenwart von ZrCl₄ in einer Blasensäulekaskade

Man leitet in zwei nach einander geschaltete, auf 160°C thermostatisierte Blasensäulen von je 100 Volumeteilen befüllt mit 141,0 Gew.-Teilen aufgeschmolzenes Phenol und 0,88 Gew.-Teile/h ZrCl₄ unter Normaldruck über 3 h von unten durch eine Gasfritte 24,8 Gew.-Teile/h auf gleicher Temperatur erhitztes Phosgen ein.

Die Reaktionsmischung der 1. Blasensäule enthaltend Chlorameisensäurephenylester und Diphenylcarbonat im Gewichtsverhältnis 2 zu 98 (Phenolumsatz 83,4%, Selektivität 98,3%) wird nach Umsetzung des vorhandenen Chlorameisensäure-phenylesters mit Phenol zu Diphenylcarbonat in einem Verweilzeitreaktor isoliert und durch Entgasung in Abgas und Sumpf aufgetrennt, der durch Destillation aufgetrennt wird.

Der am Kopf der 1. Blasensäule austretende Abgasstrom wird durch eine Gasfritte in die 2. Blasensäule geleitet.enthält In der 2. Blasensäule ist nach 3h der Phenolumsatz 22,0%.

### 1f) Phosgenierung von Phenol in Gegenwart von TiCl₄ in einer Blasensäulekaskade

Nach Durchführung der Direktphosgenierung wie beschrieben in 1e) aber mit 0,71 Gew.-Teilen TiCl₄ enthält die Reaktionsmischung in der 1. Blasensäule Chlorameisensäurephenylester und Diphenylcarbonat im Gewichtsverhältnis 0,6 zu 99,4 (Phenolumsatz 80,2%, Selektivität 99,5%). In der 2. Blasensäule ist der Phenolumsatz 12,3%.

### 1g) Phosgenierung von Phenol in Gegenwart von Ti(OC₆H₅)₄ in einer Blasensäulekaskade

Nach Durchführung der Direktphosgenierung wie beschrieben in 1e) aber mit 1,57 Gew.-Teilen Ti(OC₆H₅)₄ enthält die Reaktionsmischung in der 1. Blasensäule Chlorameisensäurephenylester und Diphenylcarbonat im Gewichtsverhältnis 0,7 zu 99,3 (Phenolumsatz 81,5%, Selektivität 99,4%). In der 2. Blasensäule ist der Phenolumsatz 17,4%.

### 1h) Phosgenierung von Phenol in Gegenwart von TiCl₄ in der Gasphase

Man dosiert von oben in eine auf 235°C thermostatisierte Rohrreaktorsapparatur mit Glaswolle unter Normaldruck 25,0 Gew.-Teile/h aufgeschmolzenes Phenol, 0,61 Gew.-Teile/h TiCl₄ und 12,5 Gew.-Teile/h auf gleicher Temperatur erhitztes Phosgen.

Das am Fuß austretende Produkt, enthaltend Chlorameisensäurephenylester und Diphenylcarbonat im Gewichtsverhältnis 0,6 zu 99,4 (Phosgenumsatz 87%, Phenolumsatz 59,8%, Selektivität 99,9%) wird nach Umsetzung des vorhandenen Chlorameisensäurephenylesters mit Phenol zu Diphenylcarbonat in einem Verweilzeitreaktor isoliert und durch Entgasung in Abgas und Sumpf aufgetrennt.

Der bei der kontinuierlichen Herstellung von Diarylcarbonaten durch Reaktion von Monophenolen und Phosgen in Gegenwart von Katalysatoren anfallende Chlorwasserstoff kann nach Entfernung der geringfügigen Phosgen- und Monophenolverunreinigungen zB. durch Ausfrieren ohne aufwendige Reinigung gegebenenfalls nach Behandlung mit Aktivkohle einer elektrochemischen Oxidation zugeführt werden.

### Beispiele 2a-b

### Rückführung von Chlorwasserstoff aus der Diphenylcarbonatherstellung durch thermische Oxidation mit Sauerstoff und Elektrolyse

### 2a) Rückführung von Chlorwasserstoff aus der Diphenylcarbonatherstellung durch thermische Oxi-dation mit Sauerstoff in Kombination mit einer Elektrolyse unter Verwendung einer Sauerstoffverzehrkathode

Ein Strom von 35,9 Gew.-Teilen/h gereinigten Chlorwasserstoff aus einer Diarylcarbonatherstellung wird in zwei Teilströme aufgeteilt. 29,5 Gew.-Teile/h werden einer HCl-Oxidation und 6,4 Gew.-Teile/h einer HCl-Absorption zugeführt. 29,5 Gew.-Teile/h HCl werden mit 12,9 Gew.-Teile/h Sauerstoff (Gehalt größer 99 %) einer katalytischen HCl-Oxidation zugeführt. Die Oxidation erfolgt bei 333°C und 3,4 bar. Der HCl-Umsatz im Reaktor beträgt 85 %. Das den Reaktor verlas-sende Gasgemisch wird auf 100°C abgekühlt, die HCl mit dem Reaktionswasser in einer HCl-Absorption kondensiert. Hierzu wird in die Salzsäure 26 aus der HCl-Absorption ein Teilstrom des aus der Elektrolyse kommenden an Salzsäure abgereichterten Anolytsäure-Stromes 28 eingeleitet. Es werden 32,1 Gew.-Teile/h der an Salzsäure abgereicherten Anolytsäure 28 mit einer HCl-Konzentration von 12,2 Gew.-% der HCl-Absorption zugeführt. Der abgekühlte Prozessgasstrom (Gewichtsverhältnis HCl, Sauerstoff, Chlor und Wasser 4,4/7,4/24,4/6,18) wird mit 6,4 Gew.-Teile/h gereinigten Chlorwasserstoff in die HCl-Absorption geleitet. In dieser HCl-Absorption-Einheit wird eine 30 Gew.%-ige Salzsäure 26 hergestellt, die mit dem Rest der abgereicherten Anolyt-säure 28 vereinigt und erneut der Elektrolysezelle zugeführt wird. 2,96 Gew.-Teile/h der abgereicherten Anolytsäure 28 werden aus dem Anolytsäure-Kreislauf ausgeschleust.

Die Elektrolyse wird mit einer Stromdichte von 5 kA/m² bei 55°C und einer Spannung von 1,39 V betrieben. Als Anoden- und Kathodenwerkstoff wird ein Palladium-stabilisiertes Titan eingesetzt. An der Rutheniumoxid-beschichteten Anode der Fa. DENORA, Deutschland, wird 10,1 Gew.-Teile/h Chlor entwickelt. Anoden- und Kathoden-Halbschale werden durch eine Ionenaustauschermembran der Fa. DUPONT, Typ Nafion 324 getrennt. Als Kathode wird eine Sauerstoffverzehrkathode der Fa. ETEK eingesetzt, die einen Rhodiumsulfid-geträgerten Katalysator enthält. Sauerstoff wird dem Kathoden-Halbelement mit 100 % Überschuss zugeführt, d.h. mit 9,17 Gew.-Teile/h. Der Sauerstoff wird in die Elektrolyse recycliert, ein Purge-Strom von 1 % der Feedmenge wird hinter der Elektrolyse 9 abgeführt (nicht gezeichnet) oder in der HCl-Oxidation 5 verwendet. Der Druck in der Anodenhalbzelle ist höher als der in der Kathodenhalbzelle. Der Differenzdruck beträgt 200 mbar. Der Kathodenhalbzelle wird ein Kondensatstrom von 8,8 Gew.-Teile/h entnommen.

Die Elektrolyseeinheit 9 besteht aus 615 Elektrolyseelementen, wobei ein Element aus einer Anodenhalbschale mit Anode, einer Ionenaustauschermembran und einer Kathodenhalbschale mit Sauerstoffverzehrkathode besteht.

### 2b) Rückführung von Chlorwasserstoff aus der Diphenylcarbonatherstellung durch thermische Oxidation mit Sauerstoff in Kombination mit einer Chloralkali-Elektrolyse

Die Oxidation wird wie in 2a) beschrieben durchgeführt.

Wie in Beispiel 1 wird ein gereinigter HCl-Gasstrom von 35,9 Gew.-Teilen/h aus einer Diarylcarbonatherstellung der thermischen HCl-Oxidation mit 100 % Sauerstoff-Überschuss, d.h. 15,7 Gew.-Teilen/h Sauerstoff, zugeführt. Der Umsatz beträgt 85 %, so dass den Reaktor 5,4 Gew.-Teile/h HCl, 9,0 Gew.-Teile/h Sauerstoff, 7,5 Gew.-Teile/h Wasser und 29,7 Gew.-Teile/h Chlor verlassen. Dieses Prozessgas 19 wird einer HCl-Absorption zugeführt, die mit einem ersten Teilstrom von 177,8 Gew.-Teilen/h aus einer NaCl-Elektrolyse stammenden an NaCl verarmten NaCl-haltigen Lösung (18,3 Gew.-% NaCl) betrieben wird. In dieser NaCl-haltigen Lösung wird das Wasser und der Chlorwasserstoff des Prozessgases 19 absorbiert. Der die Absorption verlassende Strom setzt sich wie folgt zusammen: 152,8 Gew.-Teile/h Wasser, 32,5 Gew.-Teile/h NaCl, 5,4 Gew.-Teile/h Chlorwasserstoff. Anschließend wird dieser Strom mit dem zweiten Teilstrom der NaCl-haltigen Lösung von 118,2 Gew.-Teile/h vereinigt, mit 26,4 Gew.-Teilen/h fester NaCl versetzt und wieder der NaCl-Elektrolyse zugeführt. Die NaCl-Elek-trolyse besteht aus 1475 bipolaren Elektrolyseelementen je 2,71m² Membranfläche. Die NaCl-Elektrolyse wird mit Anodenhalbschalen aus Titan, die eine Edelmetalloxid-beschichtete Titan-Anode trägt, betrieben. Die Kathodenhalbschale besteht aus Nickel und trägt eine Edelmetalloxid-beschichtete Nickel-Kathode. Anoden- und Kathodenhalbschale werden durch eine Ionenaustauschermembran der Fa. DUPONT Nafion 982 getrennt. Anodisch werden 21,2 Gew.-Teile/h Chlor, kathodisch 74,8 Gew.-Teile/h Natronlauge mit einer Konzentration von 32 Gew.-% und 0,6 Gew.-Teile/h Wasserstoff hergestellt. Der an NaCl abgereicherte Anolyt wird zum Teil wieder der HCl-Absorption zugeführt.

### Beispiel 3

### Herstellung von Polycarbonat

Aus einer Vorlage werden 8.600 Gew.-Teile/h Schmelzegemisch, bestehend aus 4.425 Gew.-Teilen/h Diphenylcarbonat, hergestellt wie beschrieben in 1a) oder 1b), und 4.175 Gew.-Teilen/h Bisphenol A, unter Zusetzen von 0,52 Gew.-Teilen/h des Phenoladdukts von Tetraphenylphosphoniumphenolat mit 65,5 % Tetraphenylphosphoniumphenolat/h gelöst in 4,5 Gew.-Teile/h Phenol/h durch einen Wärmetauscher gepumpt, auf 190°C erwärmt und durch eine Verweilkolonne bei 12 bar und 190°C geführt. Die mittlere Verweilzeit beträgt 50 Minuten.

Die Schmelze wird dann über ein Entspannungsventil in einen unter 200 mbar stehenden Abscheider geleitet. Die abfließende Schmelze wird in einem, ebenfalls unter 200 mbar stehenden, Fallfilmver-dampfer wieder auf 189°C erwärmt und in einer Vorlage aufgefangen. Nach einer Verweilzeit von 20 Minuten wird die Schmelze in die nächsten drei, gleichartig aufgebauten Stufen gepumpt. Die Bedingungen in der 2. / 3. / 4. Stufe sind 100 / 74 / 40 mbar, 218 / 251 / 276°C und 20 / 10 / 10 Minuten. Das entstandene Oligomere hat eine relative Viskosität von 1,09. Alle Brüden werden über Druckregelungen in eine unter Vakuum stehende Kolonne geführt und als Kondensate abgeleitet.

Danach wird das Oligomer in einem sich anschliessenden Korbreaktor bei 278°C und 3,0 mbar bei einer Verweilzeit von 45 Minuten zu einem höhermolekularen Produkt aufkondensiert. Die relative Viskosität beträgt 1,195. Die Brüden werden kondensiert.

Vom Schmelzestrom, der in einen weiteren Korbreaktor geleitet wird, wird mittels einer Zahnradpumpe ein Teilstrom von 150 Gew.-Teilen/h Schmelze abgezweigt, mit 0,185 Gew.-Teilen/h einer 5 %-igen wässrigen Phosphorsäure versetzt, über einen statischen Mischer mit einem Länge-zu-Durchmesser-Verhältnis von 20 gerührt und wieder in den Hautschmelzestrom zurückgeleitet. Direkt nach dem Zusammentreffen wird die Phosphorsäure im gesamten Schmelzestrom mittels eines weiteren statischen Mischers homogen verteilt.

Die so behandelte Schmelze wird in einem weiteren Korbreaktor bei 284°C, 0,7 mbar und bei einer mittleren Verweilzeit von 130 Minuten weiter den Prozessbedingungen ausgesetzt, ausgetragen und granuliert.

Die Brüden werden in der Vakuumanlage und dahinter kondensiert.

Das erhaltene Polycarbonat hat folgende Kennzahlen: relative Viskosität 1,201 / phenolische OH 255 [ppm] / DPC 71 [ppm] / BPA 6 [ppm] / Phenol 56 [ppm].

Das abdestillierte Phenol kann wieder in die Diphenylcarbonatherstellung gemäß Schritt (b), wie beschrieben in den Beispielen 1a-h), rückgeführt werden.

## Patentansprüche

1. Verfahren zur Herstellung von Diarylcarbonat umfassend folgende Schritte:
(a) Herstellung von Phosgen durch Umsetzung von Chlor mit Kohlenmonoxid,
(b) Umsetzung des gemäß Schritt (a) gebildeten Phosgens mit wenigstens einem Monophenol in Gegenwart eines Katalysators unter Bildung wenigstens eines Diarylcarbonats und von Chlorwasserstoff,
(c) Abtrennung und Aufarbeitung des in Schritt (b) gebildeten Diarylcarbonats,
(d) Abtrennung und gegebenenfalls Reinigung des gemäß Schritt (b) gebildeten Chlorwasserstoffs,
(e) thermische Oxidation wenigstens eines Teils des Chlorwasserstoffs aus (d) mit Sauerstoff zu Chlor unter Bildung von Wasser,
(f) Rückführung wenigstens eines Teils des gemäß Schritt (e) hergestellten Chlors in die Herstellung von Phosgen gemäß Schritt (a),
wobei die thermische Oxidation mit Sauerstoff in Schritt (e) unter Verwendung eines oder mehrerer Katalysatoren erfolgt und
wobei in Schritt (b) Metallsalze als homogene Katalysatoren eingesetzt werden.

2. Verfahren nach Anspruch 1, **dadurch gekennzeichnet, dass** als homogener Katalysator Titanchlorid, Zirkonchlorid, Aluminiumchlorid oder Titanphenolat eingesetzt wird.

3. Verfahren nach Anspruch 1 oder 2, **dadurch gekennzeichnet, dass** das bei der Umsetzung (e) entstehende Gasgemisch, bestehend aus den Zielprodukten Chlor und Wasser, nicht umge-setztem Chlorwasserstoff und Sauerstoff, sowie weiteren Nebenbestandteilen, insbesondere Kohlendioxid und Stickstoff,
1) zur Kondensation von Salzsäure abgekühlt
2) die entstandene wässrige Lösung von Chlorwasserstoff (Salzsäure) vom Gasgemisch abgetrennt,
3) die abgetrennte Salzsäure mindestens teilweise einer elektrochemischen Oxidation zugeführt wird, in der wenigstens ein Teil der wässrigen Salzsäure zu Chlor oxidiert,
4) das in Schritt 3) anfallende Chlorgas gegebenenfalls dem in Schritt (e) anfallenden Gasgemisch zugegeben,
5) die im Gasgemisch aus den Schritten 3) und (e) vorhandenen Reste an Wasser, insbesondere durch Wäsche mit Schwefelsäure, entfernt,
6) das entstehende chlorreiche Gasgemisch von Sauerstoff und gegebenenfalls von Nebenbestandteilen befreit wird.

4. Verfahren nach einem der Ansprüche 1 bis 3, **dadurch gekennzeichnet, dass** eine Reinigung des Chlorwasserstoffs aus Schritt (b) stattfindet, bevor dieser einer thermischen Oxidation (e) unterworfen wird.

5. Verfahren gemäß einem der Ansprüche 1 bis 4, **dadurch gekennzeichnet, dass** die Reinigung gemäß Schritt (d) des gemäß Schritt (b) gebildeten Chlorwasserstoffs eine Abtrennung von Phosgen mittels Verflüssigung umfasst.

6. Verfahren gemäß einem der Ansprüche 1 bis 5, **dadurch gekennzeichnet, dass** gemäß Schritt (d) eine Reinigung des Chlorwasserstoffs mittels Ausfrieren erfolgt.

7. Verfahren gemäß einem der Ansprüche 3 bis 6, **dadurch gekennzeichnet, dass** die Abtrennung des Chlorwasserstoffs vom Gasgemisch gemäß den Schritten 2) und 3) durch zusätzliche Wäsche des Gasgemisches mit Wasser oder verdünnten Salzsäure erfolgt.

8. Verfahren gemäß einem der Ansprüche 3 bis 7, **dadurch gekennzeichnet, dass** die elektrochemische Oxidation der wässrigen Chlorwasserstoff-Lösung gemäß Schritt 3) in einer Elektrolysezelle erfolgt, in welcher der Anodenraum und der Kathodenraum durch eine Ionenaustauschermembran getrennt sind.

9. Verfahren gemäß einem der Ansprüche 3 bis 8, **dadurch gekennzeichnet, dass** die elektrochemische Oxidation der wässrigen Chlorwasserstoff-Lösung gemäß Schritt 3) in einer Elektrolysezelle erfolgt, in welcher der Anodenraum und der Kathodenraum durch ein Diaphragma getrennt sind.

10. Verfahren gemäß einem der Ansprüche 3 bis 9, **dadurch gekennzeichnet, dass** als Kathode für die elektrochemische Oxidation gemäß Schritt 3) eine Gasdiffusionselektrode verwendet wird, die insbesondere als Sauerstoffverzehrkathode betrieben wird.

11. Verfahren gemäß Anspruch 10, **dadurch gekennzeichnet, dass** die Kathode Rhodiumsulfid enthält.

12. Verfahren gemäß einem der Ansprüche 3 bis 11, **dadurch gekennzeichnet, dass** die Salzsäure gemäß Schritt 3) zusammen mit Alkalichloriden, insbesondere mit Natriumchlorid in einer anodischen Chloralkali-Elektrolyse oxidiert wird.

13. Verfahren gemäß einem der Ansprüche 3 bis 12, **dadurch gekennzeichnet, dass** als Alkalichloridlösung eine aus der Chloralkali-Elektrolyse stammende abgereicherte Alkalichloridlösung verwendet wird.

14. Verfahren gemäß einem der Ansprüche 3 bis 13, **dadurch gekennzeichnet, dass** ein Teil des für die thermische Oxidation (e) vorgesehenen Chlorwasserstoffs abgezweigt wird, einer Absorption mit Wasser oder verdünnter Salzsäure, insbesondere verdünnter Salzsäure unterzogen wird und die entstehende, konzentrierte Salzsäure mit der Salzsäure aus Schritt 2) vereinigt und der Elektrolyse gemäß Schritt 3) zugeführt wird.

15. Verfahren gemäß einem der Ansprüche 1 bis 14, **dadurch gekennzeichnet, dass**, Chlor gemäß Schritt (a) zu Phosgen umgesetzt und das so erhaltene Phosgen zur Umsetzung mit Phenol zu Diphenylcarbonat gemäß Schritt (b) eingesetzt wird.

16. Verfahren gemäß einem der Ansprüche 1 bis 15, **dadurch gekennzeichnet, dass** es sich beim hergestellten Diarylcarbonat um Diphenylcarbonat handelt.

17. Verfahren gemäß einem der Ansprüche 1 bis 16, **dadurch gekennzeichnet, dass** Diarylcarbonat mit einem Bisphenol zum Oligo-/Polycarbonat und dem Monophenol umgesetzt und das so erhaltene Monophenol zur Umsetzung mit Phosgen zu Diphenylcarbonat gemäß Schritt (b) eingesetzt wird.

18. Verfahren gemäß einem der Ansprüche 1 bis 17, **dadurch gekennzeichnet, dass** die thermische Oxidation (e) mit einem Umsatz von 50 bis 98%, insbesondere mit einem Umsatz von 60 bis 95% betrieben wird.

## Claims

1. Process for preparing diaryl carbonate, which comprises the following steps:
(a) preparationral of phosgene by reaction of chlorine with carbon monoxide,
(b) reaction of the phosgene formed in step (a) with at least one monophenol in the presence of a catalyst to form at least one diaryl carbonate and hydrogen chloride,
(c) isolation and work-up of the diaryl carbonate formed in step (b),
(d) isolation and optionally purification of the hydrogen chloride formed in step (b),
(e) thermal oxidation of at least part of the hydrogen chloride from (d) to chlorine by means of oxygen with formation of water,
(f) recirculation of at least part of the chlorine prepared in step (e) to the preparation of phosgene in step (a),
where the thermal oxidation by means of oxygen in step (e) is carried out using one or more catalysts and
where, in step (b), metal salts are used as homogeneous catalysts.

2. Process according to Claim 1, **characterized in that** titanium chloride, zirconium chloride, aluminium chloride or titanium phenoxide is used as homogeneous catalyst.

3. Process according to Claim 1 or 2, **characterized in that** the gas mixture consisting of the target products chlorine and water, unreacted hydrogen chloride and oxygen and also further secondary constituents, in particular carbon dioxide and nitrogen, which is formed in the reaction (e) is
1) cooled to condense hydrochloric acid,
2) the resulting aqueous solution of hydrogen chloride (hydrochloric acid) is separated off from the gas mixture,
3) the hydrochloric acid which has been separated off is at least partly fed to an electrochemical oxidation in which at least part of the aqueous hydrochloric acid is oxidized to chlorine,
4) the chlorine gas obtained in step 3) is optionally added to the gas mixture obtained in step (e),
5) the residual amounts of water present in the gas mixture from steps 3) and (e) are removed, in particular by scrubbing with sulphuric acid,
6) the resulting chlorine-rich gas mixture is freed of oxygen and optionally of secondary constituents.

4. Process according to any of Claims 1 to 3, **characterized in that** a purification of the hydrogen chloride from step (b) takes place before the hydrogen chloride is subjected to a thermal oxidation (e).

5. Process according to any of Claims 1 to 4, **characterized in that** the purification as per step (d) of the hydrogen chloride formed in step (b) comprises removal of phosgene by means of liquefaction.

6. Process according to any of Claims 1 to 5, **characterized in that** a purification of the hydrogen chloride by means of freezing-out is carried out in step (d).

7. Process according to any of Claims 3 to 6, **characterized in that** the separation of the hydrogen chloride from the gas mixture in steps 2) and 3) is effected by additional scrubbing of the gas mixture with water or dilute hydrochloric acid.

8. Process according to any of Claims 3 to 7, **characterized in that** the electrochemical oxidation of the aqueous hydrogen chloride solution in step 3) is carried out in an electrolysis cell in which the anode space and the cathode space are separated by an ion-exchange membrane.

9. Process according to any of Claims 3 to 8, **characterized in that** the electrochemical oxidation of the aqueous hydrogen chloride solution in step 3) is carried out in an electrochemical cell in which the anode space and the cathode space are separated by a diaphragm.

10. Process according to any of Claims 3 to 9, **characterized in that** a gas diffusion electrode which is operated, in particular, as oxygen-consuming cathode is used as cathode for the electrochemical oxidation in step 3).

11. Process according to Claim 10, **characterized in that** the cathode contains rhodium sulphide.

12. Process according to any of Claims 3 to 11, **characterized in that** the hydrochloric acid is oxidized in step 3) together with alkali metal chlorides, in particular together with sodium chloride, in an anodic chloralkali electrolysis.

13. Process according to any of Claims 3 to 12, **characterized in that** a depleted alkali metal chloride solution from the chloralkali electrolysis is used as alkali metal chloride solution.

14. Process according to any of Claims 3 to 13, **characterized in that** part of the hydrogen chloride provided for the thermal oxidation (e) is branched off, subjected to absorption in water or dilute hydrochloric acid, in particular dilute hydrochloric acid, and the resulting concentrated solution is combined with the hydrochloric acid from step 2) and fed to the electrolysis in step 3).

15. Process according to any of Claims 1 to 14, **characterized in that** chlorine is converted into phosgene as per step (a) and the resulting phosgene is used for the reaction with phenol to form diphenyl carbonate in step (b).

16. Process according to any of Claims 1 to 15, **characterized in that** the diaryl carbonate prepared is diphenyl carbonate.

17. Process according to any of Claims 1 to 16, **characterized in that** diaryl carbonate is reacted with a bisphenol to form the oligocarbonate/polycarbonate and the monophenol and the resulting monophenol is used for the reaction with phosgene to form diphenyl carbonate in step (b)

18. Process according to any of Claims 1 to 17, **characterized in that** the thermal oxidation (e) is operated at a conversion of from 50 to 98%, in particular a conversion of from 60 to 95%.

## Revendications

1. Procédé de fabrication de carbonate de diaryle comprenant les étapes suivantes :
(a) la fabrication de phosgène par mise en réaction de chlore avec du monoxyde de carbone,
(b) la mise en réaction du phosgène formé selon l'étape (a) avec au moins un monophénol en présence d'un catalyseur avec formation d'au moins un carbonate de diaryle et de chlorure d'hydrogène,
(c) la séparation et le traitement du carbonate de diaryle formé à l'étape (b),
(d) la séparation et éventuellement la purification du chlorure d'hydrogène formé selon l'étape (b),
(e) l'oxydation thermique d'au moins une partie du chlorure d'hydrogène issu de (d) avec de l'oxygène pour former du chlore avec formation d'eau,
(f) le recyclage d'au moins une partie du chlore fabriqué selon l'étape (e) dans la fabrication de phosgène selon l'étape (a),
l'oxydation thermique avec de l'oxygène à l'étape (e) ayant lieu en utilisant un ou plusieurs catalyseurs, et des sels métalliques étant utilisés en tant que catalyseurs homogènes à l'étape (b).

2. Procédé selon la revendication 1, **caractérisé en ce que** du chlorure de titane, du chlorure de zirconium, du chlorure d'aluminium ou du phénolate de titane est utilisé en tant que catalyseur homogène.

3. Procédé selon la revendication 1 ou 2, **caractérisé en ce que** le mélange gazeux formé lors de la réaction (e) constitué par les produits cibles chlore et eau, chlorure d'hydrogène non réagi et oxygène, ainsi que d'autres constituants secondaires, notamment du dioxyde de carbone et de l'azote,
1) est refroidi pour la condensation d'acide chlorhydrique,
2) la solution aqueuse de chlorure d'hydrogène (acide chlorhydrique) formée est séparée du mélange gazeux,
3) l'acide chlorhydrique séparé est introduit au moins en partie dans une oxydation électrochimique dans laquelle au moins une partie de l'acide chlorhydrique aqueux est oxydée en chlore,
4) le chlore gazeux formé à l'étape 3) est éventuellement ajouté au mélange gazeux formé à l'étape (e),
5) les résidus d'eau présents dans le mélange gazeux des étapes 3) et (e) sont éliminés, notamment par lavage avec de l'acide sulfurique,
6) le mélange gazeux riche en chlore formé est débarrassé de l'oxygène et éventuellement des constituants secondaires.

4. Procédé selon l'une quelconque des revendications 1 à 3, **caractérisé en ce qu'**une purification du chlorure d'hydrogène de l'étape (b) a lieu avant que celui-ci ne soit soumis à une oxydation thermique (e).

5. Procédé selon l'une quelconque des revendications 1 à 4, **caractérisé en ce que** la purification selon l'étape (d) du chlorure d'hydrogène formé selon l'étape (b) comprend une séparation du phosgène par liquéfaction.

6. Procédé selon l'une quelconque des revendications 1 à 5, **caractérisé en ce qu'**une purification du chlorure d'hydrogène selon l'étape (d) a lieu par congélation.

7. Procédé selon l'une quelconque des revendications 3 à 6, **caractérisé en ce que** la séparation du chlorure d'hydrogène du mélange gazeux selon les étapes 2) et 3) a lieu par lavage supplémentaire du mélange gazeux avec de l'eau ou de l'acide chlorhydrique dilué.

8. Procédé selon l'une quelconque des revendications 3 à 7, **caractérisé en ce que** l'oxydation électrochimique de la solution aqueuse de chlorure d'hydrogène selon l'étape 3) a lieu dans une cellule d'électrolyse, dans laquelle la chambre d'anode et la chambre de cathode sont séparées par une membrane échangeuse d'ions.

9. Procédé selon l'une quelconque des revendications 3 à 8, **caractérisé en ce que** l'oxydation électrochimique de la solution aqueuse de chlorure d'hydrogène selon l'étape 3) a lieu dans une cellule d'électrolyse dans laquelle la chambre d'anode et la chambre de cathode sont séparées par un diaphragme.

10. Procédé selon l'une quelconque des revendications 3 à 9, **caractérisé en ce qu'**une électrode à diffusion de gaz, qui est notamment exploitée en tant que cathode à consommation d'oxygène, est utilisée en tant que cathode pour l'oxydation électrochimique selon l'étape 3).

11. Procédé selon la revendication 10, **caractérisé en ce que** la cathode contient du sulfure de rhodium.

12. Procédé selon l'une quelconque des revendications 3 à 11, **caractérisé en ce que** l'acide chlorhydrique est oxydé selon l'étape 3) conjointement avec des chlorures alcalins, notamment avec du chlorure de sodium dans une électrolyse chlore-alcali anodique.

13. Procédé selon l'une quelconque des revendications 3 à 12, **caractérisé en ce qu'**une solution de chlorure alcalin appauvrie issue de l'électrolyse chlore-alcali est utilisée en tant que solution de chlorure alcalin.

14. Procédé selon l'une quelconque des revendications 3 à 13, **caractérisé en ce qu'**une partie du chlorure d'hydrogène prévu pour l'oxydation thermique (e) est dérivée, soumise à une absorption avec de l'eau ou de l'acide chlorhydrique dilué, notamment de l'acide chlorhydrique dilué, et l'acide chlorhydrique concentré formé est réuni avec l'acide chlorhydrique de l'étape 2) et introduit dans l'électrolyse selon l'étape 3).

15. Procédé selon l'une quelconque des revendications 1 à 14, **caractérisé en ce que** du chlore est transformé en phosgène selon l'étape (a) et le phosgène ainsi obtenu est utilisé pour la réaction avec le phénol pour former du carbonate de diphényle selon l'étape (b).

16. Procédé selon l'une quelconque des revendications 1 à 15, **caractérisé en ce que** le carbonate de diaryle fabriqué est le carbonate de diphényle.

17. Procédé selon l'une quelconque des revendications 1 à 16, **caractérisé en ce que** le carbonate de diaryle est mis en réaction avec un bisphénol pour former l'oligo-/polycarbonate et le monophénol, et le monophénol ainsi obtenu est utilisé pour la réaction avec le phosgène pour former du carbonate de diphényle selon l'étape (b).

18. Procédé selon l'une quelconque des revendications 1 à 17, **caractérisé en ce que** l'oxydation thermique (e) est réalisée avec une conversion de 50 à 98 %, notamment avec une conversion de 60 à 95 %.
